# EUROPEAN PATENT SPECIFICATION

(11) **EP 2 423 211 B1**
(45) Date of publication and mention of the grant of the patent: **16.04.2014**
(21) Application number: 10766836.0
(22) Date of filing: 20.04.2010
(51) Int. Cl.: C07D 471/04

(54) **PROCESSES FOR PRODUCING (1S,6S)- OR (1R,6R)-CIS-2,8-DIAZABICYCLO[4.3.0]NONANE AND INTERMEDIATE THEREOF**
VERFAHREN ZUR HERSTELLUNG VON (1S,6S)- ODER (1R,6R)-CIS-2,8-DIAZABICYCLO[4.3.0]NONAN UND ZWISCHENPRODUKT
PROCÉDÉS DE PRODUCTION DE (1S,6S)- OU (1R,6R)-CIS-2,8-DIAZABICYCLO[4.3.0]NONANE ET LEUR INTERMÉDIAIRE

(30) Priority: 20.04.2009 JP 2009102213
(43) Date of publication of application: 29.02.2012
(73) Proprietor: KANEKA CORPORATION, Osaka (JP)
(72) Inventor: OHNUKI, Masatoshi, Takasago-shi Hyogo 676-8688 (JP); NISHIYAMA, Akira, Takasago-shi Hyogo 676-8688 (JP)
(74) Representative: HOFFMANN EITLE
(86) International application number: PCT/JP2010/002829
(87) International publication number: WO 2010/122774

(56) References cited:
- WO-A1-98/22437
- JP-A- 2 069 474
- JP-A- 5 271 229
- JP-A- H11 510 478

## Description

### TECHNICAL FIELD

The present invention relates to processes for producing (1S,6S)- or (1R,6R)-cis-2,8-diazabicyclo[4.3.0]nonane or the salt thereof and the intermediate thereof. The compounds are important as a raw material for a medicine, especially a raw material for producing moxifloxacin, which is an antibacterial agent.

### BACKGROUND ART

As a process for producing (1S,6S)- or (1R,6R)-cis-2,8-diazabicyclo[4.3.0]nonane, the following processes are reported:

### Process (1) (Patent Document 1 and Patent Document 2)

First, 2,3-pyridinedicarboxylic acid is sequentially reacted with acetic anhydride and benzylamine, to produce 6-benzyl-5,7-dihydro-6H-pyrrolo[3,4-b]pyridine-5,7-dione. Then, an optically active (1S,6S)-cis-8-benzyl-2,8-diazabicyclo[4.3.0]nonane is produced by reduction of the aromatic ring, reduction of the imide group, optical resolution using tartaric acid, recrystallization of the obtained salt and neutralization. Finally, the compound is subjected to hydrogenolysis;

### Process (2) (Patent Document 3)

First, 2,3-pyridinedicarboxylic acid is sequentially reacted with acetic anhydride and (S)-phenylethylamine, to produce an optically active 6-((S)-1-phenylethyl)-5,7-dihydro-6H-pyrrolo[3,4-b]pyridine-5,7-dione. After reduction of the aromatic ring and the imide group, the amino group at 2-position is protected with a t-butoxycarbonyl group. Then, the diastereomer was removed with silica gel column chromatography, to produce (1S,6S)-cis-2-(t-butoxycarbonyl)-8-((S)-1-phenylethyl)-2,8-diazabicyclo[4.3.0]nonane. Finally, the compound is subjected to hydrogenolysis.

### PRIOR ART

### PATENT DOCUMENT

Patent Document 1: JP2-69474A
Patent Document 2: WO99/58532
Patent Document 3: WO98/22437

### DISCLOSURE OF THE INVENTION

### PROBLEMS TO BE SOLVED BY THE INVENTION

The above conventional process (1) is troublesome, since crystallization have to be repeated for improving the optical purity. The above conventional process (2) is not a practically preferable for industrial production, since silica gel column chromatography is used for purification.

### MEANS FOR SOLVING THE PROBLEMS

Under the above-described circumstance, the present inventors found the following facts and completed the present invention as a result of extensive studies.
· A solid (1S,6R)- or (1R,6S)-cis-7,9-dioxo-8-substituted-2,8-diazabicyclo[4.3.0]nonane derivative having high excessive diastereomer ratio (%de) can be produced by forming a salt from a (1S,6R)- or (1R,6S)-cis-7,9-dioxo-8-substituted-2,8-diazabicyclo[4.3.0]nonane derivative and an acid, and then precipitating the solid from a solvent; and
· (1S,6S)- or (1R,6R)-cis-2,8-diazabicyclo[4.3.0]nonane can be obtained by reducing the above solid nonane derivative or other methods.

The present invention relates to a process for producing a solid (1S,6R)-cis-7,9-dioxo-8-substituted-2,8-diazabicyclo[4.3.0]nonane derivative, comprising the step of forming a salt from a (1S,6R)-cis-7,9-dioxo-8-substituted-2,8-diazabicyclo[4.3.0]nonane derivative represented by the following formula (1): 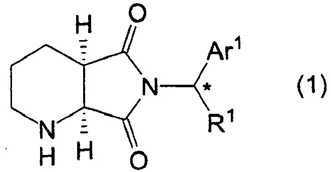
wherein * indicates an asymmetric carbon; Ar¹ is an optionally substituted C₆-₂₀ aryl group or an optionally substituted C₃₋₂₀ heteroaryl group; R¹ is an optionally substituted C₁₋₂₀ alkyl group; and wherein a (1R,6S)-cis-7,9-dioxo-8-substituted-2,8-diazabicyclo[4.3.0]nonane derivative is contained as an impurity,
with an acid represented by the following formula (2):

   H⁺A⁻ (2)
wherein A⁻ is a counter anion,
to precipitate the solid (1S,6R)-cis-7, 9-dioxo-8-substituted-2,8-diazabicyclo[4.3.0]nonane derivative represented by the following formula(3): 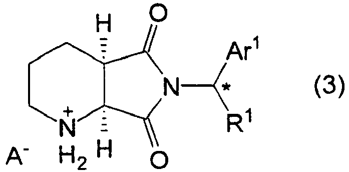
wherein *, Ar¹, R¹ and A⁻ are the same as the above.

The (1S,6R)-cis-7,9-dioxo-8-substituted-2,8-diazabicyclo[4.3.0]nonane derivative is preferably produced from 2,3-pyridinedicarboxylic acid.

The present invention also relates to a solid (1S,6R)-cis-7,9-dioxo-8-substituted-2,8-diazabicyclo[4.3.0]nonane derivative represented by the following formula (3): 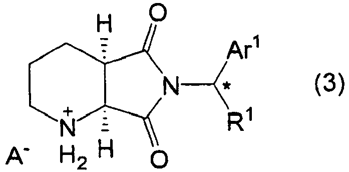
wherein * indicates an asymmetric carbon; Ar¹ is an optionally substituted C₆₋₂₀ aryl group or an optionally substituted C₃₋₂₀ heteroaryl group; R¹ is an optionally substituted C₁₋₂₀ alkyl group.

The present invention also relates to a process for producing (1S,6S)-cis-2,8-diazabicyclo[4.3.0]nonane represented by the following formula (5): 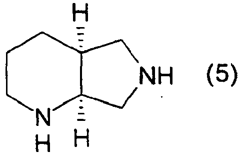 or the salt thereof, comprising the steps of
optionally neutralizing the solid (1S,6R)-cis-7,9-dioxo-8-substituted-2,8-diazabicyclo[4.3.0]nonane derivative which is represented by the following formula (3): 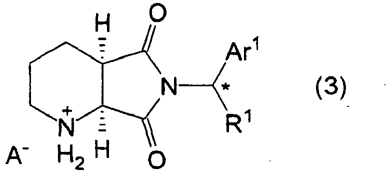
wherein *, Ar¹, R¹ and A⁻ are the same as the above,
and which is produced by the above-described process,
and then reacting a reducing agent to produce a (1S,6S)-cis-8-substituted-2,8-diazabicyclo[4.3.0]nonane derivative represented by the following formula (4): 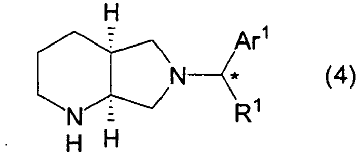
wherein *, Ar¹ and R¹ are the same as the above,
   or the salt thereof,
and subsequently reacting hydrogen in the presence of a metal catalyst to remove the substituent group on the nitrogen atom at 8-position.

The present invention also relates to a process for producing a solid (1R,6S)-cis-7,9-dioxo-8-substituted-2,8-diazabicyclo[4.3.0]nonane derivative, comprising the step of forming a salt from a (1R,6S)-cis-7,9-dioxo-8-substituted-2,8-diazabicyclo[4.3.0]nonane derivative represented by the following formula (8): 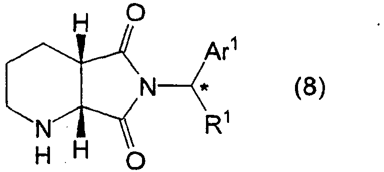
wherein * indicates an asymmetric carbon; Ar¹ is an optionally substituted C₆₋₂₀ aryl group or an optionally substituted C₃₋₂₀ heteroaryl group; R¹ is an optionally substituted C₁₋₂₀ alkyl group; and wherein a (1S,6R)-cis-7,9-dioxo-8-substituted-2,8-diazabicyclo[4.3.0]nonane derivative is contained as an impurity,
with an acid represented by the following formula (2):

   H⁺A⁻ (2)
wherein A⁻ is a counter anion,
to precipitate the solid (1R,6S)-cis-7,9-dioxo-8-substituted-2,8-diazabicyclo[4.3.0]nonane derivative represented by the following formula (9): 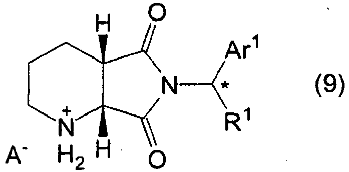
wherein *, Ar¹, R¹ and A⁻ are the same as the above.

### EFFECT OF THE INVENTION

The present invention method is practically preferable for industrial production, and (1S,6S)- or (1R,6R)-cis-2,8-diazabicyclo[4.3.0]nonane can be easily and efficiently produced according to the present invention process. In addition, the target compound can be obtained at a low price by producing a (1S,6R)- or (1R,6S)-cis-7,9-dioxo-8-substituted-2,8-diazabicyclo[4.3.0]nonane derivative from 2,3-pyridinedicarboxylic acid.

### BRIEF DESCRIPTION OF DRAWINGS

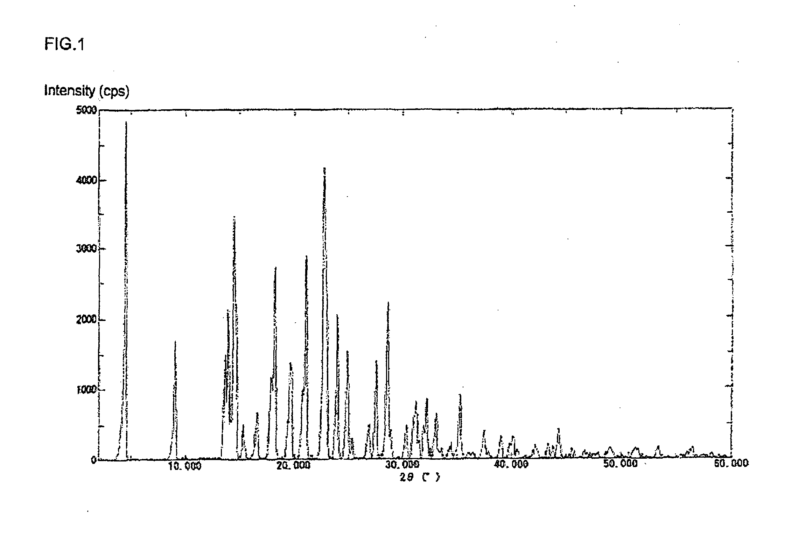

Figure 1 is the X-ray powder diffraction spectrum of (1S,6R)-cis-7,9-dioxo-8-((S)-1-phenylethyl)-2,8-diazabicyclo[4.3.0]nonane hydrochloride produced in Example 21 according to the present invention. The vertical axis represents the intensity of X-ray (cps), and the horizontal axis represents diffraction angle (2θ).

### MODE FOR CARRYING OUT THE INVENTION

Then, the present invention is specifically described by taking the production case of (1S,6S)-cis-2,8-diazabicyclo[4.3.0]nonane as an example.

The process for producing (1S,6S)-cis-2,8-diazabicyclo[4.3.0]nonane according to the present invention is represented by the following scheme.

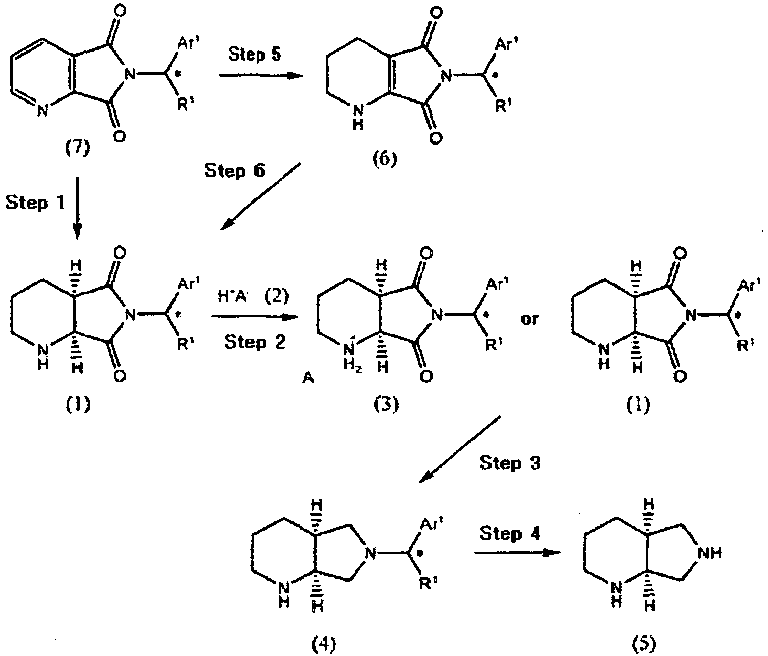

Hereinafter, each step is described in order. In the present invention, each step can be independently carried out unless otherwise described.

### Step 1

In the Step 1, a 6-substituted-5,7-dihydro-6H-pyrrolo[3,4-b]pyridine-5,7-dione derivative represented by the formula (7) is reacted with hydrogen in the presence of a metal catalyst, to reduce the aromatic ring; as a result, a (1S,6R)-cis-7,9-dioxo-8-substituted-2,8-diazabicyclo[4.3.0]nonane derivative represented by the formula (1) is produced.

In the formulae (1) and (7), * indicates an asymmetric carbon.

The Ar¹ is an optionally substituted C₆₋₂₀ aryl group or an optionally substituted C₃₋₂₀ heteroaryl group.

An aryl group is exemplified by a phenyl group, a naphthyl group, a biphenyl group and others. A heteroaryl group is exemplified by a pyridyl group, a furanyl group, a thienyl group, a pyrrolyl group, an oxazolyl group, an isooxazolyl group, a pyrazolyl group, a benzofuranyl group, a benzothiazolyl group, an indolyl group and others.

The above aryl group and heteroaryl group may be substituted with a halogen atom such as a fluorine atom, a chlorine atom, a bromine atom and an iodine atom, a nitro group, a nitroso group, a cyano group, an amino group, a hydroxyamino group, a C₁₋₁₂ alkylamino group, a C₁₋₁₂ dialkylamino group, a C₇₋₁₂ aralkylamino group, a C₇₋₁₂ diaralkylamino group, a C₁₋₁₂ alkylsulufonylamino group, a sulfonate group, a sulfonamide group, an azide group, a trifluoromethyl group, a carboxy group, a C₁₋₁₂ acyl group, a C₇₋₁₂ aroyl group, a hydroxyl group, a C₁₋₁₂ alkyloxy group, a C₇₋₁₂ aralkyloxy group, a C₆₋₁₂ aryloxy group, a C₁₋₁₂ acyloxy group, a C₇₋₁₂ aroyloxy group, a C₃₋₁₂ silyloxy group, a C₁₋₁₂ alkylsulufonyloxy group, a C₁₋₁₂ alkylthio group or other substituent group. The number of the substituent group may be not less than 0 and not more than 5.

The Ar¹ is preferably a phenyl group or a 1-naphthyl group, and more preferably a phenyl group.

The R¹ is an optionally substituted C₁₋₂₀ alkyl group. The alkyl group is specifically exemplified by a methyl group, an ethyl group, an n-propyl group, an isopropyl group, an n-butyl group, a t-butyl group and others. The number of the substituent may be not less than 0 and not more than 5. The substituent is exemplified by those exemplified in the case of Ar¹.

The R¹ is preferably a methyl group, a hydroxymethyl group, an ethyl group or an n-propyl group, more preferably a methyl group or an ethyl group, and particularly preferably a methyl group.

The compound (7) as the starting material in the Step 1 of which Ar¹ is a phenyl group, R¹ is a methyl group and an absolute configuration of the asymmetric carbon is S can be easily produced by the method described in WO98/22437 Specifically, 2,3-pyridinedicarboxylic anhydride is reacted with (S)-1-phenylethylamine and subsequently acetic anhydride.

A metal catalyst is specifically exemplified by a metal such as platinum, rhodium, palladium, nickel, cobalt, ruthenium, iridium and rhenium, the alloy thereof, the chloride thereof and others. When the metal catalyst is used, the reductive reaction can be carried out under a relative low pressure condition.

It is preferred that the catalyst is dispersed on a powdery support from the aspect of catalyst activity, repeatability, preservation stability, operability and recyclability.

A powdery support is specifically exemplified by carbon, alumina, silica-alumina, silica, barium carbonate, barium sulfate, calcium carbonate, titanium oxide, zirconium oxide, zeolite and others. A catalyst is preferably a metal, a sulfate, a hydroxide and others of platinum, rhodium or palladium supported on a powdery support.

A catalyst is specifically exemplified by platinum-carbon, platinum (II) sulfide-carbon, platinum-alumina, platinum-silica-alumina, platinum-silica, platinum-barium carbonate, platinum-barium sulfate, platinum-calcium carbonate, platinumtitanium oxide, platinum-zirconium oxide, platinum-zeolite, platinum-asbestos, platinum rhodium alloy-carbon, platinum palladium alloy-carbon, rhodium-carbon, rhodium-alumina, rhodium-silica, rhodium-calcium carbonate, palladium-carbon, palladium hydroxide (II)-carbon, palladium (II) sulfide-carbon, palladium-alumina, palladium-silica-alumina, palladium-silica, palladium-barium carbonate, palladium-barium sulfate, palladium-calcium carbonate, palladium-titanium oxide, palladium-zirconium oxide, palladium-zeolite, palladiumasbestos, ruthenium-carbon, ruthenium-alumina, rutheniumsilica, ruthenium-calcium carbonate, iridium-carbon, iridiumalumina, iridium-silica, iridium-calcium carbonate, Raney nickel and others.

A metal catalyst may be used alone, and alternatively, two or more catalysts may be used in combination.

A metal catalyst is preferably palladium-carbon, palladium hydroxide (II)-carbon, rhodium-carbon, platinum-carbon, ruthenium-carbon or Raney nickel, more preferably palladium-carbon, palladium hydroxide (II)-carbon or rhodium-carbon, and particularly preferably palladium-carbon or palladium hydroxide (II)-carbon.

The amount of a metal catalyst to be used is preferably not more than 5 times by weight, more preferably not less than 0.01 times by weight and not more than 1.0 time by weight, and particularly preferably not less than 0.05 times by weight and not more than 0.5 times by weight, relative to the compound (7), since too much metal catalyst is not preferable in terms of cost and aftertreatment.

The solvent used in the reaction is not limited as long as the solvent does not negatively affect the reaction, and is specifically exemplified by water; an alcohol solvent such as methanol, ethanol, n-propanol, isopropanol, n-butanol, tert-butanol and ethylene glycol; an ether solvent such as tetrahydrofuran, diethyl ether, 1,4-dioxane, methyl tert-butyl ether and ethylene glycol dimethyl ether; an ester solvent such as ethyl acetate, n-propyl acetate and isopropyl acetate; an aromatic hydrocarbon solvent such as benzene and toluene; a ketone solvent such as acetone and methyl ethyl ketone; an aliphatic hydrocarbon solvent such as pentane, hexane, heptane and methylcyclohexane; a halogen solvent such as methylene chloride and 1,2-dichloroethane; a sulfoxide solvent such as dimethylsulfoxide; an amide solvent such as N,N-dimethylformamide, N,N-dimethylacetamide, N,N-diethylacetamide, N-methyl-2-pyrrolidone, N-ethyl-2-pyrrolidone, N-methyl-ε-caprolactam and hexamethylphosphoramide; a urea solvent such as dimethylpropyleneurea; a phosphoric triamide solvent such as hexamethylphosphoric triamide.

Among the exemplified solvent, an alcohol solvent such as methanol, ethanol, n-propanol, isopropanol, n-butanol, tert-butanol and ethylene glycol; an ester solvent such as ethyl acetate, n-propyl acetate and isopropyl acetate; an aromatic hydrocarbon solvent such as benzene and toluene; an ether solvent such as tetrahydrofuran, diethyl ether, 1,4-dioxane, methyl tert-butyl ether and ethylene glycol dimethyl ether; a halogen solvent such as methylene chloride and 1,2-dichloroethane; an amide solvent such as N,N-dimethylformamide, N,N-dimethylacetamide, N,N-diethylacetoamide, N-methyl-2-pyrrolidone, N-ethyl-2-pyrrolidone, N-methyl-ε-caprolactam and hexamethylphosphoramide; and others are preferable.

A solvent is more preferably tetrahydrofuran, 1,4-dioxane, isopropyl ether, cyclopentyl methyl ether, dimethoxyethane, isopropanol, isopropyl acetate, N,N-dimethylacetamide or toluene, and particularly preferably tetrahydrofuran, 1,4-dioxane or isopropanol.

A solvent may be used alone, and alternatively, two or more solvents may be used in combination. When two or more solvents are used in combination, the mixing ratio is not limited.

The amount of a solvent to be used is preferably not more than 50 times by weight, and more preferably not more than 20 time by weight, relative to the compound (7), since too much solvent is not preferable in terms of cost and aftertreatment.

The reaction temperature is not limited and may be properly determined. The temperature is preferably not less than -20°C and not more than 200°C, more preferably not less than 0°C and not more than 150°C, and particularly preferably not less than 20°C and not more than 120°C, for decreasing by-product.

The pressure of hydrogen in the reaction is not limited and may be properly determined. The pressure is preferably not less than 0.3 MPa, and more preferably not less than 0.3 MPa and not more than 12 MPa.

The reaction time is not limited and may be properly determined. The reaction time is not less than 0.1 hours and not more than 48 hours, and more preferably not less than 0.5 hours and not more than 24 hours.

The order of mixing the compound (7), a solvent, a metal catalyst and hydrogen is not particularly limited, and it is preferred that hydrogen is added to the mixture of the compound (7), a solvent and a metal compound in terms of safety.

The excessive diastereomer ratio (%de) of the compound (1) obtained by the Step 1 is preferably less than 15%de, and more preferably not less than -10%de and not more than 10%de.

As an aftertreatment of the reaction, a general aftertreatment for obtaining a product from a reaction mixture may be carried out. For example, a metal catalyst is removed by filtration from the reaction mixture after the reaction and a solvent is removed from the filtrate by heating under reduced pressure, to obtain the target compound.

The thus obtained compound (1) has purity enough to be used in the following step. However, the compound (1) may be purified to further improve the purity by a general purification method such as crystallization, fractional distillation, washing the compound (1) or the salt thereof by changing solubility between an organic layer and aqueous layer, precipitating a solid from a solvent by forming a salt from the compound (1) and an acid, and column chromatography. It is preferred to precipitate a solid from a solvent by forming a salt from the compound (1) and an acid. The method is explained in the description of the Step 2.

### Step 2

In the Step 2, a solid (1S,6R)-cis-7,9-dioxo-8-substituted-2,8-diazabicyclo[4.3.0]nonane derivative represented by the formula (3) is produced by forming a salt from a (1S,6R)-cis-7,9-dioxo-8-substituted-2,8-diazabicyclo[4.3.0]nonane derivative represented by the formula (1) and an acid represented by the formula (2), to precipitate the solid. The (1S,6R)-cis-7,9-dioxo-8-substituted-2,8-diazabicyclo[4.3.0]nonane derivative (1) contains a (1R,6S)-cis-7,9-dioxo-8-substituted-2,8-diazabicyclo[4.3.0]nonane derivative as an impurity.

In the formulae (1) and (3), Ar¹, R¹ and * are the same as the above. In the formulae (2) and (3), A⁻ is a counter anion.

The compound (2) is exemplified by an optically active acid and optically inactive acid.

An optically active acid is not limited, and is specifically exemplified by tartaric acid; a diacyl tartarate derivative such as dibenzoyl tartarate, di-o-toluoyl tartarate, di-p-toluoyl tartarate, di-o-anisoyl tartarate and di-p-anisoyl tartarate; a tartaric amide derivative such as tartranilic acid, o-methyltartranilic acid, m-methyltartranilic acid, p-methyltartranilic acid, p-chlorotartranilic acid, p-nitrotartranilic acid and p-methoxytartranilic acid; malic acid; an acyl malate derivative such as benzoyl malate, p-toluoyl malate and p-chlorobenzoyl malate; a mandelic acid derivative such as mandelic acid, o-methylmandelic acid, m-methylmandelic acid, p-methylmandelic acid, p-chloromandelic acid, p-nitromandelic acid and p-methoxymandelic acid; an amino acid such as alanine, valine, phenylalanine and aspartic acid; an N-acylamino acid derivative such as N-formylalanine, N-acetylalanine, N-benzoylalanine, N-formylphenylglycine, N-acetylphenylglycine, N-benzoylphenylglycine, N-formylphenylalanine, N-acetylphenylalanine, N-benzoylphenylalanine, N-formylproline, N-acetylproline, N-benzoylproline, N-formylaspartic acid, N-acetylaspartic acid, N-benzoylaspartic acid, N-formylglutamic acid, N-acetylglutamic acid and N-benzoylglutamic acid; an N-sulfonylamino acid derivative such as N-methanesulfonylalanine, N-benzenesulfonylalanine, N-p-toluenesulfonylalanine, N-methanesulfonylphenylglycine, N-benzenesulfonylphenylglycine, N-p-toluenesulfonylphenylglycine, N-methanesulfonylproline, N-benzenesulfonylproline, N-p-toluenesulfonylproline, N-methanesulfonylphenylalanine, N-benzenesulfonylphenylalanine, N-p-toluenesulfonylphenylalanine, N-methanesulfonylaspartic acid, N-benzenesulfonylaspartic acid, N-p-toluenesulfonylaspartic acid, N-methanesulfonylglutarnic acid, N-benzenesulfonylglutamic acid and N-p-toluenesulfonylglutamic acid; lactic acid; a 2-benzyloxypropionic acid derivative such as 2-benzyloxypropionic acid and 2-(p-nitrobenzyloxy)propionic acid; a 2-aroyloxypropionic acid derivative such as 2-benzoyloxypropionic acid, 2-(p-methylbenzoyloxy)propionic acid, 2-(p-nitrobenzoyloxy)propionic acid, 2-(p-chlorobenzoyloxy)propionic acid, 2-(3,5-dichlorobenzoyloxy)propionic acid, 2-(p-methoxybenzoyloxy)propionic acid, 2-(p-tert-butylbenzoyloxy)propionic acid, 2-(1-naphthoyloxy)propionic acid and 2-(2-furoyloxy)propionic acid; a 2-aryloxypropionic acid derivative such as 2-phenoxypropionic acid; 10-camphorsulfonic acid; (+)-camphor acid; abietic acid; dehydroabietic acid; and others.

Among the exemplified acid, tartaric acid; a diacyl tartarate derivative such as dibenzoyl tartarate, di-o-toluoyl tartarate, di-p-toluoyl tartarate, di-o-anisoyl tartarate and di-p-anisoyl tartarate; and 10-camphorsulfonic acid are preferable; D-tartaric acid, L-tartaric acid and (S)-10-camphorsulfonic acid are more preferable; and D-tartaric acid and (S)-10-camphorsulfonic acid are particularly preferable.

An optically inactive acid is not limited, and specifically exemplified by an inorganic acid such as hydrogen fluoride, hydrogen chloride, hydrogen bromide, hydrogen iodide, sulfuric acid, nitric acid, phosphoric acid and boric acid; a carboxylic acid such as formic acid, acetic acid, propionic acid, butyric acid, pivalic acid, chloroacetic acid, trichloroacetic acid, trifluoroacetic acid, benzoic acid and oxalic acid; a sulfonic acid such as methanesulfonic acid, trifluoromethanesulfonic acid, benzenesulfonic acid and p-toluenesulfonic acid. Among the examples, hydrogen chloride, hydrogen bromide, sulfuric acid, acetic acid, oxalic acid, benzoic acid, methanesulfonic acid and p-toluenesulfonic acid are preferable; and hydrogen chloride, hydrogen bromide, sulfuric acid, oxalic acid and methanesulfonic acid are more preferable; and hydrogen chloride is particularly preferable.

The A⁻ of the compound (3) is preferably an optically active tartarate ion, an optically active diacyl tartarate derivative ion such as an optically active dibenzoyltartarate ion, an optically active di-o-toluoyltartarate ion, an optically active di-p-toluoyltartarate ion, an optically active di-o-anisoyltartarate ion and an optically active di-p-anisoyltartarate ion, an optically active 10-camphorsulfonate ion, a chloride ion, a bromide ion, a sulfate ion, an acetate ion, an oxalate ion, a benzoate ion, a methanesulfonate ion and a p-toluenesulfonate ion; and more preferably a D-tartarate ion, an L-tartarate ion, an (S)-10-camphorsulfonate ion, a chloride ion, a bromide ion, a sulfate ion, an oxalate ion and a methanesulfonate ion; and particularly preferably a D-tartarate ion, an (S)-10-camphorsulfonate ion and a chloride ion.

It is more preferred that an optically inactive acid is used as the acid used in the Step 2 than an optically active acid in terms of cost.

The amount of the compound (2) to be used is preferably not less than 0.1 times by mole and not more than 5 times by mole, more preferably not less than 0.1 times by mole and not more than 3 times by mole, and particularly preferably not less than 0.3 times by mole and not more than 1.5 times by mole, relative to the compound (1).

The solvent used to precipitate the solid in the Step 2 is not particularly limited, and specifically exemplified by water; an alcohol solvent such as methanol, ethanol, n-propanol, isopropanol, n-butanol, tert-butanol and ethylene glycol; an ester solvent such as ethyl acetate, n-propyl acetate and isopropyl acetate; an ether solvent such as tetrahydrofuran, diethyl ether, 1,4-dioxane, methyl tert-butyl ether, diisopropyl ether and ethylene glycol dimethyl ether; a ketone solvent such as acetone and methyl ethyl ketone; a nitrile solvent such as acetonitrile and propionitrile; an aromatic hydrocarbon solvent such as benzene, toluene and xylene; an aliphatic hydrocarbon solvent such as pentane, hexane, heptane and methylcyclohexane; a halogen solvent such as methylene chloride, 1,2-dichloroethane and chlorobenzene; a sulfoxide solvent such as dimethylsulfoxide; an amide solvent such as N,N-dimethylformamide and N,N-dimethylacetamide; a urea solvent such as dimethylpropyleneurea; a phosphoric triamide solvent such as hexamethylphosphoric triamide.

A solvent may be used alone, and alternatively, two or more solvents may be used in combination. When two or more solvents are used in combination, the mixing ratio is not limited.

A solvent is preferably water, methanol, ethanol, isopropanol, ethyl acetate, isopropyl acetate, tetrahydrofuran, methyl tert-butyl ether, diethyl ether, acetone, acetonitrile, toluene, chlorobenzene, hexane, heptane and methylcyclohexane; and more preferably water, methanol, ethanol, isopropanol and ethyl acetate; and particularly preferably water, methanol and isopropanol.

The amount of a solvent to be used is preferably not more than 50 times by weight and more preferably not more than 20 times by weight relative to the compound (1), since it is not preferable to use too much solvent in terms of cost and aftertreatment.

The method for precipitating the solid in the Step 2 is not particularly limited, and is exemplified by the followings:
Method (a) wherein the compound (1) is mixed with the acid (2) in a solvent, to precipitate the solid;
Method (b) wherein the compound (1) is mixed with the acid (2) in a solvent, and then the mixture is cooled to precipitate the solid;
Method (c) wherein the compound (1) is mixed with the acid (2) in a solvent, and then the mixture is concentrated to precipitate the solid;
Method (d) wherein the compound (1) is mixed with the acid (2) in a solvent, and then a poor solvent is added thereto to precipitate the solid;
Method (e) wherein the compound (1) is mixed with the acid (2) in a solvent, and then the solvent is substituted by a poor solvent to precipitate the solid.

The above Methods (a) to (e) may be appropriately combined to precipitate the solid. Alternatively, the acid (2) is added to the reaction mixture of the Step 1 in advance, and the compound (3) generated in the reaction mixture may be subjected to the Step 2 to precipitate the solid. When the solid is precipitated, a seed solid may be added.

The solvent to be used to precipitate the solid is exemplified by the above-described solvents. As the poor solvent used in the Method (e), toluene, hexane and others are exemplified.

The temperature to precipitate the solid in the Methods (a) to (e) is not particularly limited, and may be appropriately determined depending on the kinds of the generated salt and the used solvent. The temperature is preferably determined depending on the desired amount to be precipitated and the desired quality of the solid in the range of less than a temperature at which the compound (3) is dissolved in the used solvent or the used mixed solvent.

The compound (3) precipitated by the Methods (a) to (e) for precipitating the solid can be separated to be obtained by a means such as filtration under reduced pressure, filtration under increased pressure, centrifugation and others. When the excessive diastereomer ratio (%de) of the solid is decreased due to a mother liquid remained in the obtained solid, the solid may be further washed with an organic solvent to raise the quality if necessary.

The means to dry the solid is not particularly limited, but it is preferred to dry the solid not more than about 60°C under reduced pressure or in vacuo to avoid heat decomposition and melting.

When the excessive diastereomer ratio (%de) is not sufficiently improved, the compound (3) may be subjected to any one of the Methods (a) to (e) again, or washed with a solvent, or may be precipitated once again by a method based on the Methods (a) to (e).

The compound (3) obtained by the above method may be optionally desalted, to obtain the compound (1) with improved excessive diastereomer ratio (%de). As the method for desalting, for example, an alkaline aqueous solution is added to deionize the compound (1), the deionized compound (1) is extracted using an organic solvent, and the extraction solvent is removed by heating under reduced pressure. The base for preparing the above-described alkaline aqueous solution is exemplified by sodium hydroxide, potassium hydroxide, sodium carbonate, potassium carbonate, sodium hydrogencarbonate, potassium hydrogencarbonate and others. The above-described organic solvent for extraction is exemplified by ethyl acetate, toluene, methyl tert-butyl ether and others.

The compound (3) obtained in the Step 2 is a novel compound which is not described in any conventional documents, and it is not naturally known that the compound (3) becomes solid.

### Step 3

In the Step 3, the salt of the (1S,6S)-cis-8-substituted-2,8-diazabicyclo[4.3.0]nonane derivative represented by the formula (4) is produced by reacting the salt of the (1S,6R)-cis-7,9-dioxo-8-substituted-2,8-diazabicyclo[4.3.0]nonane derivative represented by the formula (3) with a reducing agent. For example, when it is difficult to directly use the compound (3) in the Step 3 for the reason that the compound (3) is slightly soluble in a reaction solvent and the reaction proceeds with difficulty, the compound (3) is desalted to obtain the (1S,6R)-cis-7,9-dioxo-8-substituted-2,8-diazabicyclo[4.3.0]nonane derivative represented by the formula (1) and the compound (1) is reacted with a reducing agent, to produce the (1S,6S)-cis-8-substituted-2,8-diazabicyclo[4.3.0]nonane derivative represented by the formula (4).

In the formulae (1), (3) and (4), Ar¹, R¹ and * are the same as the above.

A reducing agent is not limited, and specifically exemplified by a boron hydride compound such as diborane, borane·diethyl ether, borane·dimethylsulfide, borane·pyridine and borane·picoline; a boron hydride metal compound such as lithium borohydride, sodium borohydride, potassium borohydride, zinc borohydride, lithium triethylborohydride, sodium triethylborohydride, potassium triethylborohydride and sodium borohydride cyanide; an aluminium hydride metal compound such as lithium aluminium hydride, sodium aluminium hydride and diisobutylaluminium hydride.

Among the above examples, lithium borohydride, sodium borohydride, potassium borohydride, lithium aluminium hydride and sodium aluminium hydride are preferable; and sodium borohydride, potassium borohydride, and lithium aluminium hydride are more preferable; and sodium borohydride and lithium aluminium hydride are particularly preferable.

The amount of a reducing agent to be used is preferably not more than 10 times by mole, more preferably not less than 0.2 times by mole and not more than 10 times by mole, and particularly preferably not less than 2 times by mole and not more than 6 times by mole, relative to the compound (1) or the compound (3), since too much reducing agent is not preferable in terms of cost and aftertreatment.

An activating agent may be added as needed. Such an activating agent is not particularly limited, and specifically exemplified by aluminium chloride (III), ferric chloride (III), zinc chloride, tin chloride (IV), gallium chloride (III), sulfuric acid, boron trifluoride, iodine and others. The activating agent is preferably aluminium chloride (III) and sulfuric acid, and more preferably sulfuric acid.

The amount of an activating agent to be used is preferably not more than 5 times by mole, more preferably not less than 0.1 times by mole and not more than 5 times by mole, and particularly preferably not less than 0.5 times by mole and not more than 3 times by mole, relative to the compound (1) or the compound (3), since too much activating agent is not preferable in terms of cost and aftertreatment.

The solvent used in the Step 3 is not particularly limited as long as the solvent does not negatively affect the reaction, and specifically exemplified by an ether solvent such as tetrahydrofuran, diethyl ether, 1,4-dioxane, methyl tert-butyl ether and ethylene glycol dimethyl ether; an aromatic hydrocarbon solvent such as benzene and toluene; an aliphatic hydrocarbon solvent such as pentane, hexane, heptane and methylcyclohexane. The solvent may be used alone, and alternatively, two or more solvents may be used in combination. When two or more solvents are used in combination, the mixing ratio is not limited.

Among the exemplified solvent, tetrahydrofuran, diethyl ether, 1,4-dioxane, methyl tert-butyl ether, toluene, hexane, heptane and methylcyclohexane are preferable, and tetrahydrofuran, diethyl ether, methyl tert-butyl ether, toluene and hexane are more preferable, and tetrahydrofuran and toluene are particularly preferable.

The amount of a solvent to be used is preferably not more than 50 times by weight and more preferably not more than 20 times by weight relative to the compound (1) or the compound (3), since too much solvent is not preferable in terms of cost and aftertreatment.

The reaction temperature of the Step 3 is not limited, and may be appropriately determined. The temperature is not less than -20°C and not more than 120°C, more preferably not less than -10°C and not more than 100°C, and particularly preferably not less than 0°C and not more than 80°C, for decreasing the generation of by-product.

The reaction time is not limited, and may be appropriately determined. The reaction time is preferably not less than 0.1 hours and not more than 48 hours, and more preferably not less than 0.5 hours and not more than 24 hours.

The order of mixing the compound (1) or the compound (3), a solvent, a reducing agent and an activating agent is not particularly limited.

As an aftertreatment of the reaction, a general aftertreatment for obtaining a product from a reaction mixture may be carried out. For example, the target compound is extracted by adding water and a general extraction solvent to the reaction mixture after the reaction. The extraction solvent is exemplified by ethyl acetate, diethyl ether, methylene chloride, toluene, hexane and others. The target compound can be obtained by removing the reaction solvent and the extraction solvent from the obtained extract by heating under reduced pressure and other procedure.

The thus obtained target compound has purity enough to be used in the following step. However, the target compound may be purified to further improve the purity by a general purification method such as crystallization, fractional distillation, washing the compound (4) or the salt thereof by changing solubility between an organic layer and aqueous layer, precipitating a solid from a solvent by forming a salt from the compound (4) and an acid, and column chromatography.

### Step 4

In the Step 4, the (1S,6S)-cis-2,8-diazabicyclo[4.3.0]nonane represented by the formula (5) or the salt thereof is produced by removing (deprotecting) the substituent (the protective group) on the nitrogen atom of the (1S,6S)-cis-8-substituted-2,8-diazabicyclo[4.3.0]nonane derivative represented by the formula (4) or the salt thereof.

The method for removing the protective group is not particularly limited, but, for example, it is preferably that the compound (4) is reacted with hydrogen in the presence of a metal catalyst.

A metal catalyst is exemplified by those used in the Step 1.

The amount of a metal catalyst to be used is preferably not more than 5 times by weight, more preferably not less than 0.01 times by weight and not more than 1.0 time by weight, and particularly preferably not less than 0.05 times by weight and not more than 0.5 times by weight, relative to the compound (4), since too much metal catalyst is not preferable in terms of cost and aftertreatment.

The solvent used in the Step 4 is not limited as long as the solvent does not negatively affect the reaction, and is specifically exemplified by water; an alcohol solvent such as methanol, ethanol, n-propanol, isopropanol, n-butanol, tert-butanol and ethylene glycol; an ether solvent such as tetrahydrofuran, diethyl ether, 1,4-dioxane, methyl tert-butyl ether and ethylene glycol dimethyl ether; an ester solvent such as ethyl acetate, n-propyl acetate and isopropyl acetate; an aromatic hydrocarbon solvent such as benzene and toluene; an aliphatic hydrocarbon solvent such as pentane, hexane, heptane and methylcyclohexane.

Among the exemplified solvent, water, methanol, ethanol, isopropanol, ethyl acetate, tetrahydrofuran and toluene are preferable; and water, methanol, ethanol, isopropanol, ethyl acetate and toluene are more preferable; and isopropanol is particularly preferable. A solvent may be used alone, and alternatively, two or more solvents may be used in combination. When two or more solvents are used in combination, the mixing ratio is not limited.

The amount of a solvent to be used is preferably not more than 50 times by weight, and more preferably not more than 20 time by weight, relative to the compound (4), since too much solvent is not preferable in terms of cost and aftertreatment.

The reaction temperature is not limited and may be properly determined. The temperature is preferably not less than -20°C and not more than 120°C, more preferably not less than 0°C and not more than 100°C, and particularly preferably not less than 20°C and not more than 80°C, for decreasing by-product.

The pressure of hydrogen is not particularly limited and may be properly determined. The pressure is preferably not more than 3 MPa, and more preferably not less than 0.01 MPa and not more than 1.0 MPa.

The order of mixing the compound (4), a solvent, a metal catalyst and hydrogen is not particularly limited. It is preferred that hydrogen is added to the mixture of the compound (4), a metal catalyst and a solvent in terms of safety.

As an aftertreatment of the reaction, a general aftertreatment for obtaining a product from a reaction mixture may be carried out. For example, a catalyst is removed by filtration from the reaction mixture after the reaction, and the target compound can be obtained from the filtrate by removing a reaction solvent by heating under reduced pressure and other procedure, to obtain the target compound.

The thus obtained compound (5) has purity enough to be used in the following step. However, the compound (5) may be purified to further improve the purity by a general purification method such as crystallization, fractional distillation, precipitating a solid by forming a salt from the compound (5) and an acid, and column chromatography. The purification method is preferably fractional distillation.

### Step 5

In the Step 5, the 6-substituted-2,3,4,5,6,7-hexahydro-1H-pyrrolo[3,4-b]pyridine-5,7-dione derivative represented by the formula (6) or the salt thereof is produced by reacting 6-substituted-5,7-dihydro-6H-pyrrolo[3,4-b]pyridine-5,7-dione represented by the formula (7) with hydrogen in the presence of a metal catalyst.

The Ar¹, R¹ and * are the same as the above.

The compound (6) is a novel compound which is not described in any conventional documents.

The metal catalyst used in the Step 1 can be used as the metal catalyst in the Step 5.

The amount of a metal catalyst is preferably not more than 5 times by weight, more preferably not less than 0.01 times by weight and not more than 1.0 time by weight, and particularly preferably not less than 0.05 times by weight and not more than 0.5 times by weight, relative to the compound (7), since too much amount of the metal catalyst is not preferable in terms of cost and aftertreatment.

As the solvent used in the Step 5, those used in the Step 1 can be used.

The amount of a solvent to be used is preferably not more than 50 times by weight and more preferably not more than 20 times by weight relative to the compound (7), since too much amount of the solvent is not preferable in terms of cost and aftertreatment.

The reaction temperature is not limited and may be properly determined. The temperature is preferably not less than -20°C and not more than 150°C, more preferably not less than 0°C and not more than 120°C, particularly preferably not less than 20°C and not more than 100°C, and even more preferably not less than 50°C and not more than 80°C.

The pressure of hydrogen is not particularly limited and may be properly determined. The pressure is preferably less than 0.3 MPa, more preferably not less than 0.001 MPa and not more than 0.3 MPa, particularly preferably not less than 0.001 MPa and not more than 0.2 MPa, and even more preferably not less than 0.001 MPa and not more than 0.1 MPa.

The reaction time is not limited, and is preferably not less than 0.1 hours and not more than 48 hours, and more preferably not less than 0.5 hours and not more than 24 hours.

The order of adding the compound (7), a solvent, a metal catalyst and hydrogen is not particularly limited, it is preferred that hydrogen is added to the mixture of the compound (7), a metal catalyst and a solvent in terms of safety.

As an aftertreatment of the reaction, a general aftertreatment for obtaining a product from a reaction mixture may be carried out. For example, a metal catalyst is removed by filtration from a reaction mixture after the reaction and a solvent is removed from the filtrate with heating under reduced pressure or other procedure, to obtain the target compound.

The thus obtained compound (6) has purity enough to be used in the following step. However, the compound (6) may be purified to further improve the purity by a general purification method such as crystallization, fractional distillation, washing the compound (6) or the salt thereof by changing solubility between an organic layer and aqueous layer, precipitating a solid by forming a salt from the compound (6) and an acid, and column chromatography. The purification method is preferably column chromatography.

### Step 6

In the Step 6, the 6-substituted-2,3,4,5,6,7-hexahydro-1H-pyrrolo[3,4-b]pyridine-5,7-dione derivative represented by the formula (6) or the salt thereof is reacted with hydrogen in the presence of a metal catalyst, to produce the (1S,6R)-cis-7,9-dioxo-8-substituted-2,8-diazabicyclo[4.3.0]nonane derivative represented by the formula (1).

The Ar¹, R¹ and * are the same as the above.

The metal catalyst used in the Step 1 can be similarly used in the Step 6.

The amount of a metal catalyst to be used is preferably not more than 5 times by weight, more preferably not less than 0.01 times by weight and not more than 1.0 time by weight, and particularly preferably not less than 0.05 times by weight and not more than 0.5 times by weight, relative to the compound (6), since too much metal catalyst is not preferable in terms of cost and aftertreatment.

The solvent used in the Step 1 can be similarly used in the Step 6.

The amount of a solvent to be used is preferably not more than 50 times by weight and more preferably not more than 20 times by weight relative to the compound (6), since too much solvent is not preferable in terms of cost and aftertreatment.

The reaction temperature is not limited and may be properly determined. The temperature is preferably not less than -20°C and not more than 120°C, more preferably not less than 0°C and not more than 100°C, and particularly preferably not less than 20°C and not more than 80°C, for decreasing by-product.

The pressure of hydrogen is not particularly limited and may be properly determined. The pressure is preferably not more than 3 MPa, and more preferably not less than 0.01 MPa and not more than 1.0 MPa.

The reaction time is not limited, and is preferably not less than 0.1 hours and not more than 48 hours, and more preferably not less than 0.5 hours and not more than 24 hours.

The order of mixing the compound (6), a solvent, a metal catalyst and hydrogen is not particularly limited, and it is preferred that hydrogen is added to the mixture of the compound (6), a metal catalyst and a solvent in terms of safety.

The excessive diastereomer ratio (%de) of the compound (1) obtained in the Step 6 is preferably not less than 15%de, more preferably not less than 35%de, and particularly preferably not less than 50%de.

The Step 5 and the Step 6 may be continuously carried out.

As an aftertreatment of the reaction, a general aftertreatment for obtaining a product from a reaction mixture may be carried out. For example, the target compound can be obtained by removing a metal catalyst with filtration from a reaction mixture after the reaction and removing a solvent from the filtrate with heating under reduced pressure or other procedure.

The thus obtained compound (1) has purity enough to be used in the following step. However, the compound (1) may be purified to further improve the purity by a general purification method such as crystallization, fractional distillation, washing the compound (1) or the salt thereof by changing solubility between an organic layer and aqueous layer, precipitating a solid by forming a salt from the compound (1) and an acid, and column chromatography. It is preferred to precipitate a solid by forming a salt from the compound (1) and an acid. The method is explained in the description of Step 2.

It is possible with reference to the process described in WO98/22437 (Patent Document 3) to produce (1R,6R)-cis-2,8-diazabicyclo[4.3.0]nonane having the configuration opposite to the (1S,6S)-cis-2,8-diazabicyclo[4.3.0]nonane produced by the present invention. Specifically, a 6-substituted-5,7-dihydro-6H-pyrrolo[3,4-b]pyridine-5,7-dione derivative is produced from 2,3-pyridinedicarboxylic anhydride and (R)-1-phenylethylamine. The above target compound can be produced using the obtained 6-substituted-5,7-dihydro-6H-pyrrolo[3,4-b]pyridine-5,7-dione derivative as a starting material through a (1R,6S)-cis-7,9-dioxo-8-substituted-2,8-diazabicyclo[4.3.0]nonane derivative or the salt thereof and a (1R,6R)-cis-8-substituted-2,8-diazabicyclo[4.3.0]nonane derivative or the salt thereof with reference to the above method. The embodiment is also included in the present invention range.

In the present invention, the term "solid" means a crystal which has a symmetric structure or a regular structure, an amorphous material, and the mixture of a crystal and an amorphous material. All of the aspects of a solid are included in the present invention range.

### EXAMPLES

Hereinafter, the present invention is described in more detail with Examples; however, it is not intended that the present invention is not restricted by the Examples in any way.

The purity and the diastereomer purity (= excessive diastereomer ratio (%de)) of a cis-7,9-dioxo-8-substituted-2,8-diazabicyclo[4.3.0]nonane derivative, a cis-8-substituted-2,8-diazabicyclo[4.3.0]nonane derivative and a 6-substituted-2,3,4,5,6,7-hexahydro-1H-pyrrolo[3,4-b]pyridine-5,7-dione derivative which are described in the Examples were measured by the HPLC with the following condition. The purity of cis-2,8-diazabicyclo[4.3.0]nonane was measured by an internal reference method using ¹H-NMR, and the optical purity thereof was measured by the HPLC with the following condition. In the internal reference method, methyl benzoate was used as an internal reference.

The term "wt%" and "area%" represent purity, and "wt%" means % by weight and "area%" means the percentage of an area.

### [Analysis methods for chemical purity and content with gradient condition]

Column: COSMOSIL 5C18ARII 250 x 4.6 mm manufactured by NACALAI TESQUE, INC.
Mobile phase A (%) / Mobile phase B (%) = 90 / 10 to 40 / 60
Mobile phase A: 0.1% phosphoric acid aqueous solution
Mobile phase B: acetonitrile
Flow rate: 1.0 ml/min
Detection means: UV 210 nm
Column temperature: 40°C
Retention time:
   6-(((S)-1-phenylethyl)-5,7-dihydro-6H-pyrrolo[3,4-b]pyridine-5,7-dione: 16.0 minutes
   cis-7,9-dioxo-8-((S)-1-phenylethyl)-2,8-diazabicyclo[4.3.0]nonane: 9.1 minutes
   cis-8-((S)-1-phenylethyl)-2,8-diazabicyclo[4.3.0]nonane: 2.4 minutes
   6-(((S)-1-(1-naphthalenyl))-5,7-dihydro-6H-pyrrolo[3,4-b]pyridine-5,7-dione: 19.1 minutes
   cis-8-((S)-1-(1-naphthalenyl))-2,8-diazabicyclo[4.3.0]nonane: 10.5 minutes
   6-(((S)-1-phenylethyl)-2,3,4,5,6,7-hexahydro-1H-pyrrolo[3,4-b]pyridine-5,7-dione: 17.1 minutes

### [Analysis method for diastereomer purity with isocratic condition]

Column: CHIRALCEL AD-H 250 x 4.6 mm manufactured by DAICEL CHEMICAL INDUSTRIES, LTD.
Mobile phase: hexane / isopropanol = 9 / 1 by volume
Flow rate: 1.0 ml/min
Detection means: UV 210 nm
Column temperature: 30°C
Retention time:
   (1S,6R)-cis-7,9-dioxo-8-((S)-1-phenylethyl)-2,8-diazabicyclo[4.3.0]nonane: 13.6 minutes
   (1R,6S)-cis-7,9-dioxo-8-((S)-1-phenylethyl)-2,8-diazabicyclo[4.3.0]nonane: 17.9 minutes
   (1S,6R)-cis-8-((S)-1-(1-naphthalenyl))-2,8-diazabicyclo[4.3.0]nonane: 12.5 minutes
   (1R,6S)-cis-8-((S)-1-(1-naphthalenyl))-2,8-diazabicyclo[4.3.0]nonane: 16.4 minutes

### [Analysis method for optical purity with isocratic condition]

In the method, the amino groups at 2- and 8-position of (1S,6S)-2,8-diazabicyclo[4.3.0]nonane were protected with t-butoxycarbonyl groups, and then, the measurement was carried out.
Column: CHIRALCEL AD-H 250 x 4.6 mm manufactured by DAICEL CHEMICAL INDUSTRIES, LTD.
Mobile phase: hexane / isopropanol = 98 / 2 by volume
Flow rate: 1.0 ml/min
Detection means: UV 210 nm
Column temperature: 25°C
Retention time:
   (1S,6S)-2,8-diazabicyclo[4.3.0]nonane protected at 2- and 8-position with t-butoxycarbonyl groups: 23.3 minutes
   (1R,6R)-2,8-diazabicyclo[4.3.0]nonane protected at 2- and 8-position with t-butoxycarbonyl groups: 29.1 minutes

### Reference Example 1: Production of 6-((S)-1-phenylethyl)-5,7-dihydro-6H-pyrrolo[3,4-b]pyridine-5,7-dione

Acetic anhydride (17 ml, 2.6 equivalents) was added to 2,3-pyridinedicarboxylic acid (10.00 g, 60 mmol), and the mixture was stirred at 110°C for 4 hours. The reaction mixture was concentrated under reduced pressure. After tetrahydrofuran (60 ml) was added to the obtained concentrate, (S)-1-phenylethylamine (7.27 g, 1.0 equivalent) was added thereto dropwide at 25°C. After the mixture was stirred at 25°C for 2 hours, the reaction mixture was concentrated under reduced pressure. Acetic anhydride (29.40 g, 4.8 equivalents) and sodium acetate (592 mg, 0.1 equivalents) were added thereto, and the mixture was stirred at 100°C for 1.5 hours. The mixture was cooled to 5°C, and then a solution consisting of water (120 g) and ethanol (12.0 g) was added thereto dropwise, to precipitate a solid. After the mixture was stirred at 5°C for 30 minutes, the solid was filtered under reduced pressure. The solid was washed with a solution consisting of water (10 ml) and ethanol (10 ml) and dried in vacuo, to obtain the target compound as a white solid (12.55 g, purity (chemical purity): 99.6 area%, yield: 81%).

The target compound:
¹H-NMR (CDCl₃): δ (ppm) 1.95(d, 3H), 5.64(m, 1H), 7.27(dd, 1H), 7.33(t, 2H), 7.52(d, 2H), 7.58(dd, 1H), 8.10(d, 1H), 8.94(d, 1H)

### Reference Example 2: Production of 6-((S)-1-(1-naphthalenyl))-5,7-dihydro-6H-pyrrolo[3,4-b]pyridine-5,7-dione

Acetic anhydride (1.4 ml, 2.5 equivalents) was added to 2,3-pyridinedicarboxylic acid (836 mg, 5 mmol), and the mixture was stirred at 110°C for 4 hours. The reaction mixture was concentrated under reduced pressure. After tetrahydrofuran (5 ml) was added to the obtained concentrate, (S)-1-naphthylethylamine (856 mg, 1.0 equivalent) was added thereto dropwise at 20°C. The mixture was stirred at 20°C for 2 hours, and then concentrated under reduced pressure. Acetic anhydride (2.45 g, 4.4 equivalents) and sodium acetate (51 mg, 0.1 equivalents) were added thereto, and the mixture was stirred at 100°C for 1.5 hours. The mixture was cooled to 20°C, and then a solution consisting of water (10.0 g) and ethanol (1.00 g) was added dropwise. The settled out oil was separated. Ethanol (2.00 g) was added to the oil, to precipitate a solid. After the mixture was stirred at 20°C for 3 hours, the solid was filtered under reduced pressure. The solid was washed with ethanol (2 ml) and dried in vacuo, to obtain the target compound as a pale pink solid (1.14 g, purity: 98.0 area%, yield: 74%).

The target compound:
¹H-NMR (CDCl₃): δ(ppm) 2.06(d, 3H), 6.39(m, 1H), 7.43-7.55(m, 4H), 7.82(d, 1H), 7.84(d, 1H), 8.02(d, 1H), 8.04(d, 1H), 8.17(d, 1H), 8.90(d, 1H)

### Example 1: Production of (1S,6R)-cis-7,9-dioxo-8-((S)-1-phenylethyl)-2,8-diazabicyclo[4.3.0]nonane

The solution consisting of 6-((S)-1-phenylethyl)-5,7-dihydro-6H-pyrrolo[3,4-b]pyridine-5,7-dione produced in Reference Example 1 (99.64 g, 392 mmol), isopropanol (784 ml), 10 wt% palladium hydroxide (II) - carbon (9.96 g) was stirred under a hydrogen gas atmosphere of ambient pressure at 70°C for 18 hours. The catalyst was separated by filtration under reduced pressure. The filtrate was concentrated under reduced pressure and the concentrate was dried in vacuo, to obtain the target compound as a yellow oil (106.19 g, yield: 93%, purity: 98.3 area%, diastereomer purity: 17.5%de).

The target compound:
¹H-NMR (CDCl₃): δ (ppm) 1.50(m, 2H), 1.57(m, 1H), 1.76(br, 1H), 1.81(d, 3H), 1.91(m, 1H), 2.65(m, 1H), 2.76-2.84(m, 2H), 3.76(d, 1H), 5.39(m, 1H), 7.26(t, 1H), 7.32(t, 2H), 7.42(d, 2H)

### Example 2: Production of (1S,6R)-cis-7,9-dioxo-8-((S)-1-phenylethyl)-2,8-diazabicyclo[4.3.0]nonane hydrochloride

The crude (1S,6R)-cis-7,9-dioxo-8-((S)-1-phenylethyl)-2,8-diazabicyclo[4.3.0]nonane produced in Example 1 (290 mg, purity: 98.3 area%, diastereomer purity: 17.5 %de, 1 mmol) was dissolved in isopropanol (2.30 g). To the solution, 30 wt% hydrogen chloride in isopropanol (111 mg, 0.9 equivalents) was added, to precipitate a solid. After the mixture was stirred at 20°C for 14 hours, the solid was separated by filtration under reduced pressure. The solid was washed with ethyl acetate (2 ml) and dried in vacuo, to obtain the target compound as a white solid (155 mg, purity: 99.9 area%, diastereomer purity: 95.3%de, yield: 52%). The content of (1S,6R)-cis-7,9-dioxo-8-((S)-1-phenylethyl)-2,8-diazabicyclo[4.3.0]nonane included in 290 mg of the crude compound was 148 mg, and 128 mg of the target compound was recovered in a solid (recovery ratio: 87%).

The target compound:
¹H-NMR (D₂O): δ(ppm) 1.45(m, 1H), 1.77(m, 2H), 1.79(d, 3H), 2.18(m, 1H), 2.96(m, 1H), 3.30(m, 2H), 4.62(d, 1H), 5.46(m, 1H), 7.35.-7.46(m, 5H)

### Example 3: Production of (1S,6R)-cis-7,9-dioxo-8-((S)-1-naphthalenyl)-2,8-diazabicyclo[4.3.0]nonane hydrochloride

The solution consisting of 6-((S)-1-naphthalenyl)-5,7-dihydro-6H-pyrrolo[3,4-b]pyridine-5,7-dione produced in Reference Example 2 (310 mg, 1 mmol), tetrahydrofuran (2 ml) and 5 wt% palladium hydroxide (II) - carbon (62 mg) was reacted under 0.5 MPa of hydrogen atmosphere at 90°C for 72 hours (yield: 53%, diastereomer purity: 5.6%de).

The catalyst was separated by filtration under reduced pressure. The filtrate was concentrated under reduced pressure, and the obtained concentrate was dissolved in isopropanol (2.78 g). To the solution, 30 wt% hydrogen chloride in isopropanol (126 mg, 1.0 equivalent) was added dropwise at 20°C, to precipitate a solid. The mixture was stirred at 40°C for 30 minutes and further at 20°C for 14 hours. The solid was separated by filtration under reduced pressure and washed with ethyl acetate (2 ml).

Isopropanol (2.78 g) was added to the solid, and the mixture was stirred at 40°C for 1 hour and further at 20°C for 1 hour. The solid was separated by filtration under reduced pressure. The solid was sequentially washed with isopropanol (0.5 ml) and ethyl acetate (2 ml), and dried in vacuo, to obtain the target compound as a white solid (79 mg, purity: 90.0 area%, diastereomer purity: 97.8%de, yield: 21%).

The target compound:
¹H-NMR (D₂O): δ(ppm) 1.08(m, 1H), 1.60(m, 2H), 1.90(d, 3H), 1.99(m, 1H), 2.85(m, 1H), 3.22(m, 1H), 3.31(m, 1H), 4.58(d, 1H), 6.14(m, 1H), 7.59(m, 3H), 7.88(m, 2H), 7.97 (m, 2H)

### Example 4: Production of (1S,6R)-cis-7,9-dioxo-8-((S)-1-phenylethyl)-2,8-diazabicyclo[4.3.0]nonane hydrobromide

The crude (1S,6R)-cis-7,9-dioxo-8-((S)-1-phenylethyl)-2,8-diazabicyclo[4.3.0]nonane produced in Example 1 (290 mg, purity: 98.3 area%, diastereomer purity: 17.5%de, 1 mmol) was dissolved in isopropanol (2.30 g). To the solution, 47 wt% hydrobromic acid (155 mg, 0.9 equivalents) was added dropwise, to precipitate a solid. After the mixture was stirred at 20°C for 4 hours, the solid was separated by filtration under reduced pressure. The solid was washed with ethyl acetate (2 ml) and dried in vacuo, to obtain the target compound as a white solid (104 mg, purity: 99.9 area%, diastereomer purity: 98.7%de, yield: 32%). The content of (1S,6R)-cis-7,9-dioxo-8-((S)-1-phenylethyl)-2,8-diazabicyclo[4.3.0]nonane included in 290 mg of the crude compound was 148 mg, and 79 mg of the target compound was recovered in a solid (recovery ratio: 54%).

The target compound:
¹H-NMR (D₂O): δ(ppm) 1.44(m, 1H), 1.75(m, 2H), 1.78(d, 3H), 2.17(m, 1H), 2.97(m, 1H), 3.31(m, 2H), 4.64(d, 1H), 5.47(m, 1H), 7.35-7.46 (m, 5H)

### Example 5: Production of (1S,6R)-cis-7,9-dioxo-8-((S)-1-phenylethyl)-2,8-diazabicyclo[4.3.0]nonane sulfate

The crude (1S,6R)-cis-7,9-dioxo-8-((S)-1-phenylethyl)-2,8-diazabicyclo[4.3.0]nonane produced in Example 1 (290 mg, purity: 98.3 area%, diastereomer purity: 17.5%de, 1 mmol) was dissolved in ethanol (2.76 g). Sulfuric acid (98 mg, 1.0 equivalent) was added dropwise thereto, to precipitate a solid. After the mixture was stirred at 20°C for 19 hours, the solid was separated by filtration under reduced pressure. The solid was washed with isopropanol (2 ml) and dried in vacuo, to obtain the target compound as a white solid (94 mg, purity: 99.7 area%, diastereomer purity: 92.8%de, yield: 27%). The content of (1S,6R)-cis-7,9-dioxo-8-((S)-1-phenylethyl)-2,8-diazabicyclo[4.3.0]nonane included in 290 mg of the crude compound was 148 mg, and 66 mg of the target compound was recovered in a solid (recovery ratio: 45%).

The target compound:
¹H-NMR (D₂O): δ(ppm) 1.44(m, 1H), 1.77(m, 2H), 1.78(d, 3H), 2.18(m, 1H), 3.00(m, 1H), 3.35(m, 2H), 4.68(d, 1H), 5.46(m, 1H), 7.35-7.46(m, 5H)

### Example 6: Production of (1S,6R)-cis-7,9-dioxo-8-((S)-1-phenylethyl)-2,8-diazabicyclo[4.3.0]nonane methanesulfonate

The crude (1S,6R)-cis-7,9-dioxo-8-((S)-1-phenylethyl)-2,8-diazabicyclo[4.3.0]nonane produced in Example 1 (290 mg, purity: 98.3 area%, diastereomer purity: 17.5%de, 1 mmol) was dissolved in isopropanol (2.30 g). Methanesulfonic acid (86 mg, 0.9 equivalents) was added thereto, to precipitate a solid. After the mixture was stirred at 20°C for 2 hours, the solid was separated by filtration under reduced pressure. The solid was washed with ethyl acetate (2 ml) and dried in vacuo, to obtain the target compound as a white solid (160 mg, purity: 99.9 area%, diastereomer purity: 97.2%de, yield : 45%). The content of (1S,6R)-cis-7,9-dioxo-8-((S)-1-phenylethyl)-2,8-diazabicyclo[4.3.0]nonane included in 290 mg of the crude compound was 148 mg, and 111 mg of the target compound was recovered in a solid (recovery ratio: 75%).

The target compound:
¹H-NMR (D₂O): δ(ppm) 1.44(m, 1H), 1.75(m, 2H), 1.79 (d, 3H), 2.17(m, 1H), 2.80(S, 3H), 2.95(m, 1H), 3.30(m, 2H), 4.60(d, 1H), 5.46(m, 1H), 7.35-7.44(m, 5H)

### Example 7: Production of (1S,6R)-cis-7,9-dioxo-8-((S)-1-phenylethyl)-2,8-diazabicyclo[4.3.0]nonane oxalate

The crude (1S,6R)-cis-7,9-dioxo-8-((S)-1-phenylethyl)-2,8-diazabicyclo[4.3.0]nonane produced in Example 1 (290 mg, purity: 98.3 area%, diastereomer purity: 17.5%de, 1 mmol) was dissolved in isopropanol (9.20 g). Oxalic acid (90 mg, 1.0 equivalent) was added dropwise thereto, to precipitate a solid. After the mixture was stirred at 20°C for 2 hours, the solid was separated by filtration under reduced pressure. The solid was washed with ethyl acetate (2 ml). Methanol (5.53 g) was added to the obtained solid, and the mixture was stirred at 20°C for 3 hours. The solid was separated by filtration under reduced pressure. Then, the solid was washed with isopropanol (2 ml) and dried in vacuo, to obtain the target compound as a white solid (113 mg, purity: 99.8 area%, diastereomer purity: 98. 4%de, yield: 42%). The content of (1S,6R)-cis-7,9-dioxo-8-((S)-1-phenylethyl)-2,8-diazabicyclo[4.3.0]nonane included in 290 mg of the crude compound was 148 mg, and 105 mg of the target compound was recovered in a solid (recovery ratio: 71%).

The target compound:
¹H-NMR (D₂O): δ(ppm) 1.44(m, 1H), 1.73(m, 2H), 1.79(d, 3H), 2.15(m, 1H), 2.90(m, 1H), 3.25(m, 2H), 4.54(d, 1H), 5.46(m, 1H), 7.35-7.46(m, 5H)

### Example 8: Production of (1S,6R)-cis-7,9-dioxo-8-((S)-1-phenylethyl)-2,8-diazabicyclo[4.3.0]nonane D-tartarate

The crude (1S,6R)-cis-7,9-dioxo-8-((S)-1-phenylethyl)-2,8-diazabicyclo[4.3.0]nonane produced in Example 1 (290 mg, purity: 98.3 area%, diastereomer purity: 17.5%de, 1 mmol) was dissolved in a solution consisting of methanol (2.88 g) and water (2.88 g). D-Tartaric acid (150 mg, 1.0 equivalent) was added thereto, to precipitate a solid. After the mixture was stirred at 20°C for 19 hours, the solid was separated by filtration under reduced pressure. The solid was washed with methanol (2 ml) and dried in vacuo, to obtain the target compound as a white solid (231 mg, purity: 99.6 area%, diastereomer purity: 96.7%de, yield: 61%). The content of (1S,6R)-cis-7,9-dioxo-8-((S)-1-phenylethyl)-2,8-diazabicyclo[4.3.0]nonane included in 290 mg of the crude compound was 148 mg, and 148 mg of the target compound was recovered in a solid (recovery ratio: 100%).

The target compound:
¹H-NMR (D₂O): δ(pom) 1.44(m, 1H), 1.77(m, 2H), 1.79(d, 3H), 2.18(m, 1H), 3.00(m, 1H), 3.34(m, 2H), 4.45(d, 2H), 4.68(d, 1H), 5.46(m, 1H), 7.35-7.45(m, 5H)

### Example 9: Production of (1S,6R)-cis-7,9-dioxo-8-((S)-1-phenylethyl)-2,8-diazabicyclo[4.3.0]nonane L-tartarate

The crude (1S,6R)-cis-7,9-dioxo-8-((S)-1-phenylethyl)-2,8-diazabicyclo[4.3.0]nonane produced in Example 1 (290 mg, purity: 98.3 area%, diastereomer purity: 17.5%de, 1 mmol) was dissolved in a solution consisting of methanol (2.88 g) and water (2.88 g). L-Tartaric acid (150 mg, 1.0 equivalent) was added thereto, to precipitate a solid. After the mixture was stirred at 20°C for 19 hours, the solid was separated by filtration under reduced pressure. The solid was washed with methanol (2 ml) and dried in vacuo, to obtain the target compound as a white solid (246 mg, purity: 99.7 area%, diastereomer purity: 38.5%de, yield : 66%).

The target compound:
¹H-NMR (D₂O): δ(ppm) 1.44(m, 1H), 1.77(m, 2H), 1.79(d, 3H), 2.18(m, 1H), 3.00(m, 1H), 3.34(m, 2H), 4.45(d, 2H), 4.68(d, 1H), 5.46(m, 1H), 7.35-7.45(m, 5H)

Example 10: Production of (1S,6R)-cis-7,9-dioxo-8-((S)-1-phenylethyl)-2,8-diazabicyclo[4.3.0]nonane (S)-camphorsulfonate

The crude (1S,6R)-cis-7,9-dioxo-8-((S)-1-phenylethyl)-2,8-diazabicyclo[4.3.0]nonane produced in Example 1 (95.57 g, purity: 98.3 area%, diastereomer purity: 17.5%de, 329 mmol) was dissolved in ethyl acetate (329 ml). (S)-Camphorsulfonic acid (30.51 g, 0.4 equivalents) was added thereto, to precipitate a solid. After the mixture was stirred at 20°C for 1 hour, the solid was separated by filtration under reduced pressure.

To the obtained solid, a solution consisting of ethyl acetate (329 ml) and isopropanol (9.56 g) was added. The mixture was stirred at 60°C for 30 minutes and further at 20°C for 30 minutes. The solid was separated by filtration under reduced pressure. To the solid, a solution consisting of ethyl acetate (329 ml) and isopropanol (9.56 g) was added again. The mixture was stirred at 60°C for 30 minutes and further at 20°C for 30 minutes. The solid was separated by filtration under reduced pressure. The solid was washed with ethyl acetate (55 ml) and dried in vacuo, to obtain the target compound as a white solid (48.03 g, diastereomer purity: 98.1%de, yield : 30%).

The content of (1S,6R)-cis-7,9-dioxo-8-((S)-1-phenylethyl)-2,8-diazabicyclo[4.3.0]nonane included in 95.57 g of the crude compound was 49.90 g, and 24.95 g of the target compound was recovered in a solid (recovery ratio: 50%).

The target compound:
¹H-NMR (D₂O): δ(ppm) 0.83(s, 3H), 1.04(s, 3H), 1.44(m, 2H), 1.64(m, 1H), 1.77(m, 2H), 1.78(d, 3H), 1.98(d, 1H), 2.05(m, 1H), 2.16(m, 1H), 2.18(m, 1H), 2.37-2.45(m, 2H), 2.86(d, 1H), 2.97(m, 1H), 3.28(d, 1H), 3.31(m, 2H), 4.63(d, 1H), 5.46(m, 1H), 7.35-7.44(m, 5H)

### Example 11: Production of (1S,6R)-cis-7,9-dioxo-8-((S)-1-phenylethyl)-2,8-diazabicyclo[4.3.0]nonane

To (1S,6R)-cis-7,9-dioxo-8-((S)-1-phenylethyl)-2,8-diazabicyclo[4.3.0]nonane (S)-camphorsulfonate produced in Example 10 (48.03 g, diastereomer purity: 98.1%de, 97.9 mmol), toluene (165 ml), water (82 ml) and 28 wt% aqueous ammonia (15.96 g, 0.8 equivalents) were added, in order to extract the target compound in the organic layer. After the obtained organic layer was washed with water (82 ml) twice, the organic layer was concentrated under reduced pressure and dried in vacuo, to obtain the target compound as a pale yellow oil (26.04 g, yield: 97%, purity: 98.2 area%, diastereomer purity: 98.1%de).

### Example 12: Production of (1S,6S)-cis-8-((S)-1-phenylethyl)-2,8-diazabicyclo[4.3.0]nonane

Lithium aluminium hydride (573 mg, 6.0 equivalents) was added to tetrahydrofuran (15 ml). To the mixture, the solution consisting of (1S,6R)-cis-7,9-dioxo-8-((S)-1-phenylethyl)-2,8-diazabicyclo[4.3.0]nonane (673 mg, 2.5 mmol) produced by the method described in Example 11 and tetrahydrofuran (2.5 ml) was added dropwise at 5°C. The mixture was stirred under reflux for 15 hours, and then, cooled to 5°C. To the mixture, a solution consisting of water (0.57 ml) and tetrahydrofuran (8.4 ml), a 30 wt% sodium hydroxide aqueous solution (0.19 ml) and water (1 ml) were sequentially added dropwise. The insoluble matter was separated by filtration and washed with tetrahydrofuran (10 ml). The obtained filtrate was concentrated under reduced pressure and dried in vacuo, to obtain the target compound as a pale orange-colored oil (541 mg, purity: 48.1 area%, yield: 53%).

The target compound:
¹H-NMR (CDCl₃): δ(ppm) 1.20(d, 3H), 1.40(m, 1H), 1.50(m, 1H), 1.55(br, 1H), 1.65(m, 2H), 2.11(m, 1H), 2.42(dd, 1H), 2.49-2.57(m, 2H), 2.69-2.78(m, 2H), 2.94(dt, 1H), 3.15(m, 1H), 3.39(m, 1H), 7.28-7.35(m, 5H)

### Example 13: Production of (1S,6S)-cis-8-((S)-1-phenylethyl)-2,8-diazabicyclo[4.3.0]nonane

The (1S,6R)-cis-7,9-dioxo-8-((S)-1-phenylethyl)-2,8-diazabicyclo[4.3.0]nonane produced in Example 10 (1.09 g, 4.0 mmol) was dissolved in tetrahydrofuran (6.52 g), and sodium borohydride (636 mg, 4.2 equivalents) was added thereto. Subsequently, sulfuric acid (433 mg, 1.1 equivalents) was added dropwise thereto at 5°C. The mixture was stirred under reflux for 2 hours and further at 50°C for 16 hours. The reaction mixture was concentrated under reduced pressure, and then, water (5.01 g) and concentrated hydrochloric acid (1.82 g) were added dropwise. The mixture was stirred under reflux for 4 hours. After the reaction mixture was concentrated to 12.99 g under reduced pressure, 30 wt% sodium hydroxide aqueous solution (2.33 g, 4.4 equivalents) and toluene (7.2 ml) were added thereto to extract the target compound in the organic layer. The organic layer was washed with water (7.2 ml) twice, and concentrated under reduced pressure and dried in vacuo, to obtain the target compound as a pale yellow oil (443 mg, purity: 79.7 wt%, yield: 38%).

### Example 14: Production of (1S,6S)-cis-2,8-diazabicyclo[4.3.0]nonane

The solution consisting of (1S,6S)-cis-8-((S)-1-phenylethyl)-2,8-diazabicyclo[4.3.0]nonane produced in Example 12 (488 mg, 1.2 mmol), isopropanol (4.9 ml) and 10wt% palladium hydroxide (II) - carbon (49 mg) was stirred under hydrogen atmosphere at 70°C for 13 hours. After the catalyst was removed by filtration under reduced pressure, the filtrate was concentrated under reduced pressure. Water (2 ml) was added to the concentrate. The aqueous layer was washed with toluene (4 ml) twice. The aqueous layer was concentrated under reduced pressure and the concentrate was dried in vacuo, to obtain the target compound as a pale brown oil (146 mg, yield: 65%, purity: 66.8wt%, diastereomer purity: 92.5%de).

The target compound:
¹H-NMR (CDCl₃): δ(ppm) 1.44(m, 1H), 1.55(m, 1H), 1.70(m, 2H), 1.88(br, 2H), 2.09(m, 1H), 2.61(dt, 1H), 2.78(d, 1H), 2.90-3.04(m, 4H), 3.17(m, 1H)

### Comparative Example 1: Production of cis-7,9-dioxo-8-((S)-1-phenylethyl)-2,8-diazabicyclo[4.3.0]nonane (according to Example 1b of WO98/22437)

The solution consisting of 6-((S)-1-phenylethyl)-5,7-dihydro-6H-pyrrolo[3,4-b]pyridine-5,7-dione (252 mg, 1 mmol), 2-methoxyethanol (2 ml) and 10wt% palladium - carbon (25 mg) was stirred under 0.4 MPa of hydrogen atmosphere at 100°C for 22 hours. After the catalyst was removed by filtration under reduced pressure, the filtrate was concentrated under the reduced pressure and the concentrate was dried in vacuo, to obtain the target compound as a pale brown oil (252 mg, purity: 98.0 area%, yield: 89%, diastereomer purity: 0.0%de).

### Comparative Example 2: Production of cis-7,9-dioxo-8-((S)-1-phenylethyl)-2,8-diazabicyclo[4.3.0]nonane (according to Example E of JP2003-48890A)

The solution consisting of 6-((S)-1-phenylethyl)-5,7-dihydro-6H-pyrrolo[3,4-b]pyridine-5,7-dione (252 mg, 1 mmol), tetrahydrofuran (2 ml) and 5wt% palladium - carbon (32 mg) was stirred under 10 MPa of hydrogen atmosphere at 90°C for 24 hours. After the catalyst was removed by filtration under reduced pressure, the filtrate was concentrated under reduced pressure and the concentrate was dried in vacuo, to obtain the target compound as a pale brown oil (265 mg, purity: 98.0 area%, yield: 90%, diastereomer purity: -0.1%de).

### Example 15: Production of (1S,6R)-6-((S)-1-phenylethyl)-2,3,4,5,6,7-hexahydro-1H-pyrrolo[3,4-b]pyridine-5,7-dione

The solution consisting of 6-((S)-1-phenylethyl)-5,7-dihydro-6H-pyrrolo[3,4-b]pyridine-5,7-dione produced by the method of Reference Example 1 (756 mg, 3 mmol), tetrahydrofuran (6 ml) and 10 wt% palladium - carbon (76 mg) was stirred under a hydrogen gas atmosphere of ambient pressure at 66°C for 24 hours. After the catalyst was removed by filtration under reduced pressure, the filtrate was concentrated under reduced pressure.

The obtained concentrate was purified with column chromatography (packing material: silica gel, mobile phase: ethyl acetate). The fraction containing the target compound was concentrated under reduced pressure and the concentrate was dried in vacuo, to obtain the target compound as a yellow oil (324 mg, purity: 98.0 area%, yield: 42%).

The target compound:
¹H-NMR (CDCl₃):δ (ppm) 1.79(d, 3H), 1.84(m, 2H), 2.31(t, 2H), 3.34(m, 2H), 4.96(br, 1H), 5.29(m, 1H), 7.23(d, 1H), 7.30(t, 2H), 7.42 (d, 2H)

### Example 16: Production of (1S, 6R) -cis-7,9-dioxo-8-((S)-1-phenylethyl)-2,8-diazabicyclo[4.3.0]nonane

The solution consisting of 6-((S)-1-phenylethyl)-2,3,4,5,6,7-hexahydro-1H-pyrrolo[3,4-b]pyridine-5,7-dione produced in Example 15 (296 mg, 1.1 mmol), tetrahydrofuran (2.2 ml) and 10wt% palladium - carbon (30 mg) was stirred under a hydrogen gas atmosphere of ambient pressure at 70°C for 24 hours. After the catalyst was removed by filtration under reduced pressure, the content of the target compound in the filtrate was measured (yield: 93%, diastereomer purity: 62.7%de).

### Example 17: Production of (1S,6R)-cis-7,9-dioxo-8-((S)-1-phenylethyl)-2,8-diazabicyclo[4.3.0]nonane

The solution of 6-((S)-1-phenylethyl)-5,7-dihydro-6H-pyrrolo[3,4-b]pyridine-5,7-dione produced by the method of Reference Example 1 (2.54 g, 10 mmol), tetrahydrofuran (10 ml) and 5wt% palladium hydroxide (II) - carbon (254 mg) was stirred under a hydrogen gas atmosphere of ambient pressure at 66°C for 24 hours. After the catalyst was removed by filtration under reduced pressure, the filtrate was concentrated under reduced pressure and the concentrate was dried in vacuo, to obtain the target compound as a yellow oil (2.69 g, purity: 99.0 area%, yield: 94%, diastereomer purity: 44.3%de).

With respect to the production process of (1S,6R)-cis-7,9-dioxo-8-((S)-1-phenylethyl)-2,8-diazabicyclo[4.3.0]nonane, Examples 15 to 17 of the present invention were compared with Comparative Examples 1 and 2 as prior arts. As a result, the diastereomer purity of the target compound in Comparative Examples 1 and 2 was very low as -0.1 to 0.0%de. On the other hand, it was possible in Examples 15 and 16 corresponding the step 5 and step 6 of the present invention to produce the target compound with high diastereomer purity as 62.7%de.

In Example 17, the step 5 and step 6 of the present invention were continuously carried out without removing the metal catalyst by filtration in the Step 5. Nevertheless, the target compound having diastereomer purity of 44.3%de could be produced with yield of 94% which was similar to the yields of Comparative Examples 1 and 2.

It was demonstrated from the above results that (1S,6R)-cis-7,9-dioxo-8-((S)-1-phenylethyl)-2,8-diazabicyclo[4.3.0]nonane having high diastereomer purity could be efficiently produced according to the present invention.

### Example 18: Production of (1S,6R)-cis-7,9-dioxo-8-((S)-1-phenylethyl)-2,8-diazabicyclo[4.3.0]nonane hydrochloride

The crude (1S,6R)-cis-7,9-dioxo-8-((S)-1-phenylethyl)-2,8-diazabicyclo[4.3.0]nonane produced in Example 17 (287 mg, purity: 99.0 area%, diastereomer purity: 44.3%de, 1 mmol) was dissolved in isopropanol (2.30 g). To the solution, 30wt% hydrogen chloride in isopropanol (136 mg, 1.1 equivalents) was added dropwise to precipitate a solid. After the mixture was stirred at 20°C for 2 hours, the solid was separated by filtration under reduced pressure. The solid was washed with ethyl acetate (2 ml) and dried in vacuo, to obtain the target compound as a white solid (204 mg, purity: 99.9 area%, diastereomer purity: 97.0%de, yield: 69%).

The content of (1S,6R)-cis-7,9-dioxo-8-((S)-1-phenylethyl)-2,8-diazabicyclo[4.3.0]nonane included in 287 mg of the crude compound was 182 mg, and 172 mg of the target compound was recovered in a solid (recovery ratio: 94%).

### Example 19: Production of (1S,6R)-cis-7,9-dioxo-8-((S)-1-phenylethyl)-2,8-diazabicyclo[4.3.0]nonane hydrochloride

The crude (1S,6R)-cis-7,9-dioxo-8-((S)-1-phenylethyl)-2,8-diazabicyclo[4.3.0]nonane produced in Example 17 (287 mg, purity: 99.0 area%, diastereomer purity: 44.3%de, 1 mmol) was dissolved in isopropanol (2.30 g). To the solution, concentrated hydrochloric acid (114 mg, 1.1 equivalents) was added dropwise to precipitate a solid. After the mixture was stirred at 20°C for 4 hours, the solid was separated by filtration under reduced pressure. The solid was washed with ethyl acetate (2 ml) and dried in vacuo, to obtain the target compound as a white solid (206 mg, purity: 99.9 area%, diastereomer purity: 98.0%de, yield: 67%).

The content of (1S,6R)-cis-7,9-dioxo-8-((S)-1-phenylethyl)-2,8-diazabicyclo[4.3.0]nonane included in 287 mg of the crude compound was 182 mg, and 168 mg of the target compound was recovered in a solid (recovery ratio: 92%).

### Example 20: Production of (1S,6R)-cis-7,9-dioxo-8-((S)-1-phenylethyl)-2,8-diazabicyclo[4.3.0]nonane (S)-camphorsulfonate

The crude (1S,6R)-cis-7,9-dioxo-8-((S)-1-phenylethyl)-2,8-diazabicyclo[4.3.0]nonane produced in Example 17 (287 mg, purity: 99.0 area%, diastereomer purity: 44.3%de, 1 mmol) was dissolved in a solution consisting of isopropanol (1.15 g) and ethyl acetate (1.15 g). To the solution, (S)-camphorsulfonic acid (174 mg, 0.75 equivalents) was added dropwise to precipitate a solid. After the mixture was stirred at 20°C for 2 hours, the solid was separated by filtration under reduced pressure. The solid was washed with ethyl acetate (2 ml) and dried in vacuo, to obtain the target compound as a white solid (285 mg, purity: 98.8 area%, diastereomer purity: 98.6%de, yield: 55%).

The content of (1S,6R)-cis-7,9-dioxo-8-((S)-1-phenylethyl)-2,8-diazabicyclo[4.3.0]nonane included in 287 mg of the crude compound was 182 mg, and 139 mg of the target compound was recovered in a solid (recovery ratio: 76%).

### Example 21: X-ray diffraction spectrum of (1S,6R)-cis-7,9-dioxo-8-((S)-1-phenylethyl)-2,8-diazabicyclo[4.3.0]nonane hydrochloride

The X-ray diffraction spectrum of (1S,6R)-cis-7,9-dioxo-8-((S)-1-phenylethyl)-2,8-diazabicyclo[4.3.0]nonane hydrochloride produced by the method of Example 19 is shown as Figure 1.

In the XRD, specific peaks were shown at about 4.5°, 9.0°, 13.6°, 13.9°, 14.5°, 14.7°, 16.7°, 17.9°, 18.2°, 19.6°, 20.9°, 21.1°, 22.8°, 23.9°, 24.9°, 27.5°, 28.6°, 31.2°, 32.2°, 33.1° and 35.2° in degrees 2θ. In particular, significant peaks were shown at about 4.5°, 9.0°, 13.9°, 14.5°, 18.2°, 19.6°, 21.1°, 22.8°, 23.9°, 24.9°, 27.5° and 28.6° in degrees 2θ, and most significant peaks were shown at about 4.5°, 14.5°, 18.2°, 21.1° and 22.8° in degrees 2θ. From the result, it was found that the solid had a crystal structure.
X-ray powder diffractometer: MiniFlex-11 manufactured by Rigaku corporation
Measurement condition: CuKα1 ray
   Tube voltage: 30 kV
   Tube current: 15 mA

## Claims

1. A process for producing a solid (1S,6R)-cis-7,9-dioxo-8-substituted-2,8-diazabicyclo[4.3.0]nonane derivative, comprising the step of forming a salt from a (1S,6R)-cis-7,9-dioxo-8-substituted-2,8-diazabicyclo[4.3.0]nonane derivative represented by the following formula (1): 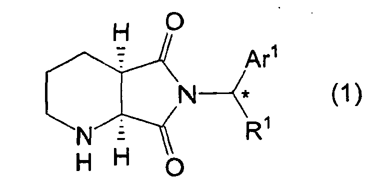
wherein * indicates an asymmetric carbon; Ar¹ is an optionally substituted C₆₋₂₀ aryl group or an optionally substituted C₃₋₂₀ heteroaryl group; R¹ is an optionally substituted C₁₋₂₀. alkyl group; and wherein a (1R,6S)-cis-7,9-dioxo-8-substituted-2,8-diazabicyclo[4.3.0]nonane derivative is contained as an impurity,
with an acid represented by the following formula (2):
H⁺A⁻ (2)
wherein A⁻ is a counter anion,
to precipitate the solid (1S,6R)-cis-7,9-dioxo-8-substituted-2,8-diazabicyclo[4.3.0]nonane derivative represented by the following formula(3): 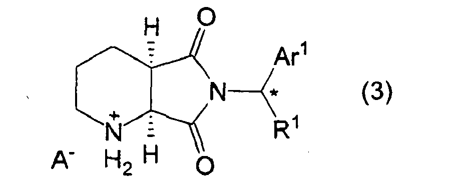
wherein *, Ar¹, R¹ and A⁻ are the same as the above.

2. The production process according to claim 1, wherein Ar¹ is a phenyl group, R¹ is a methyl group, and an absolute configuration of the asymmetric carbon is S, in the substituent group on the nitrogen atom at 8-position.

3. The production process according to claim 1 or 2, wherein the acid (2) is hydrogen chloride, sulfuric acid, hydrogen bromide, methanesulfonic acid or oxalic acid.

4. The production process according to claim 1 or 2, wherein the acid (2) is (S)-10-camphorsulfonic acid or D-tartaric acid.

5. The production process according to any one of claims 1 to 4, wherein the (1S,6R)-cis-7,9-dioxo-8-substituted-2,8-diazabicyclo[4.3.0]nonane derivative represented by the formula (1) is produced by reacting a 6-substituted-2,3,4,5,6,7-hexahydro-1H-pyrrolo[3,4-b]pyridine-5,7-dione derivative represented by the following formula (6): 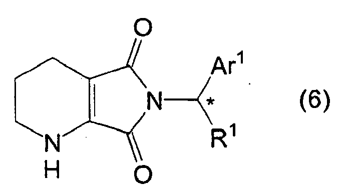
wherein * indicates an asymmetric carbon; Ar¹ is a phenyl group; R¹ is a methyl group,
or the salt thereof with hydrogen in the presence of a metal catalyst.

6. A process for producing (1S,6S)-cis-2,8-diazabicyclo[4.3.0]nonane represented by the following formula (5): 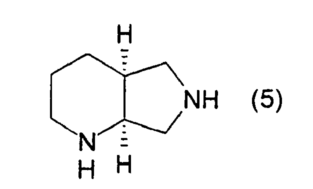 or the salt thereof, comprising the steps of
optionally neutralizing the solid (1S,6R)-cis-7,9-dioxo-8-substituted-2,8-diazabicyclo[4.3.0]nonane derivative represented by the following formula (3): 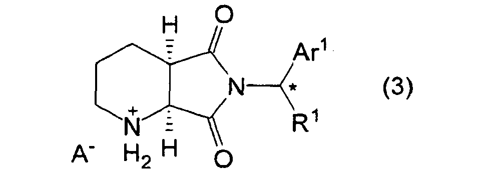
wherein *, Ar¹, R¹ and A⁻ are the same as the above, produced by the method according to any one of claims 1 to 5,
and then reacting a reducing agent to produce a (1S,6S)-cis-8-substituted-2,8-diazabicyclo[4.3.0]nonane derivative represented by the following formula (4): 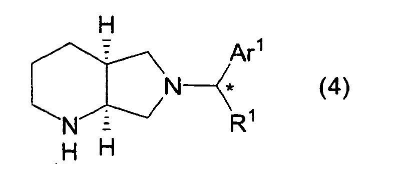
wherein *, Ar¹ and R¹ are the same as the above, or the salt thereof,
and subsequently reacting hydrogen in the presence of a metal catalyst to remove the substituent group on the nitrogen atom at 8-position.

7. A solid (1S,6R)-cis-7,9-dioxo-8-substituted-2,8-diazabicyclo[4.3.0]nonane derivative represented by the following formula (3): 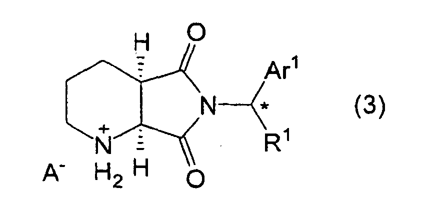
wherein * indicates an asymmetric carbon; Ar¹ is an optionally substituted C₆₋₂₀ aryl group or an optionally substituted C₃₋₂₀ heteroaryl group; R¹ is an optionally substituted C₁₋₂₀ alkyl group.

8. The solid according to claim 7, wherein Ar¹ is a phenyl group, R¹ is a methyl group, and an absolute configuration of the asymmetric carbon is S, in the substituent group on the nitrogen atom at 8-position.

9. The solid according to claim 7 or 8, wherein A⁻ is a chloride ion, a hydrogensulfate ion, a bromide ion, a methanesulfonate ion or an oxalate ion.

10. The solid according to claim 7 or 8, wherein A⁻ is an (S)-10-camphorsulfonate ion or a D-tartarate ion.

11. The solid according to claim 7, wherein A⁻ is a chloride ion and the solid exhibits an x-ray powder diffraction pattern having specific peaks at 4.5°, 14.5°, 18.2°, 21.1° and 22.8° in degrees 2θ.

12. The solid according to claim 7, wherein A⁻ is a chloride ion and the solid exhibits an x-ray powder diffraction pattern having specific peaks at 4.5°, 9.0°, 13.9°, 14.5°, 18.2°, 19.6°, 21.1°, 22.8°, 23.9°, 24.9°, 27.5° and 28.6° in degrees 2θ.

13. A 6-substituted-2,3,4,5,6,7-hexahydro-1H-pyrrolo[3,4-b]pyridine-5,7-dione derivative represented by the following formula (6): 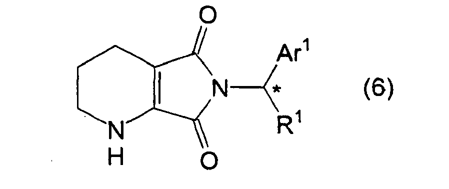
wherein * indicates an asymmetric carbon; Ar¹ is a phenyl group; R¹ is a methyl group,
or the salt thereof.

14. A process for producing a solid (1R,6S)-cis-7,9-dioxo-8-substituted-2,8-diazabicyclo[4.3.0]nonane derivative, comprising the step of forming a salt from a (1R,6S)-cis-7,9-dioxo-8-substituted-2,8-diazabicyclo[4.3.0]nonane derivative represented by the following formula (8): 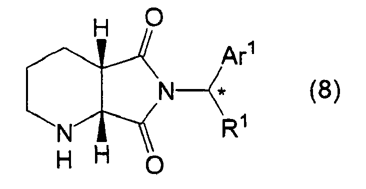
wherein * indicates an asymmetric carbon; Ar¹ is an optionally substituted C₆₋₂₀ aryl group or an optionally substituted C₃₋₂₀ heteroaryl group; R¹ is an optionally substituted C₁₋₂₀ alkyl group; and wherein a (1S,6R)-cis-7,9-dioxo-8-substituted-2,8-diazabicyclo[4.3.0]nonane derivative is contained as an impurity,
with an acid represented by the following formula (2):
H⁺A⁻ (2)
wherein A⁻ is a counter anion,
to precipitate the solid (1R,6S)-cis-7,9-dioxo-8-substituted-2,8-diazabicyclo[4.3.0]nonane derivative represented by the following formula (9): 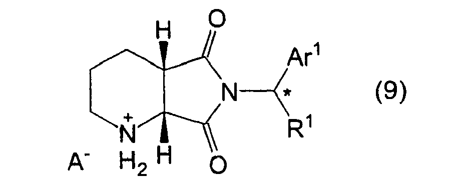
wherein *, Ar¹, R¹ and A⁻ are the same as the above.

## Patentansprüche

1. Verfahren zur Herstellung eines festen 8-substituierten (1S,6R)-cis-7,9-Dioxo-2,8-diazabicyclo[4.3.0]nonan-Derivats, umfassend einen Schritt, in dem aus einem 8-substituierten (1S,6R)-cis-7,9-Dioxo-2,8-diazabicyclo[4.3.0]nonan-Derivat der nachstehenden Formel (1): 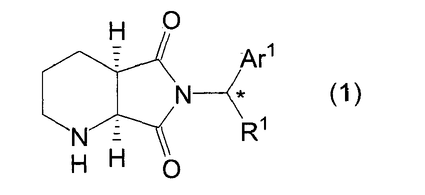
worin * einen asymmetrischen Kohlenstoff bezeichnet; Ar¹ eine gegebenenfalls substituierte C₆₋₂₀-Arylgruppe oder eine gegebenenfalls substituierte C₃₋₂₀-Heteroarylgruppe ist; R¹ eine gegebenenfalls substituierte C₁₋₂₀-Alkylgruppe ist; und worin ein 8-substituiertes (1R,6S)-cis-7,9-Dioxo-2,8-diazabicyclo[4.3.0]nonan-Derivat als Verunreinigung enthalten ist,
mit einer Säure der nachstehenden Formel (2):
H⁺A⁻ (2)
worin A⁻ ein Gegenanion ist,
ein Salz gebildet wird, um das feste 8-substituierte (1S,6R)-cis-7,9-Dioxo-2,8-diazabicyclo[4.3.0]nonan-Derivat der nachstehenden Formel (3) auszufällen: 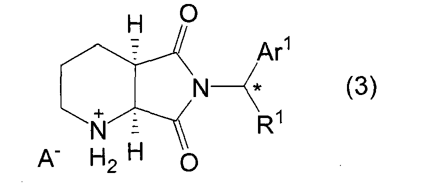
worin *, Ar¹, R¹ und A⁻ die gleichen wie oben sind.

2. Herstellungsverfahren gemäss Anspruch 1, wobei Ar¹ eine Phenylgruppe ist, R¹ eine Methylgruppe ist und die absolute Konfiguration des asymmetrischen Kohlenstoffs in der Substituentengruppe am Stickstoffatom in der 8-Position S ist.

3. Herstellungsverfahren gemäss Anspruch 1 oder 2, wobei die Säure (2) Chlorwasserstoff, Schwefelsäure, Bromwasserstoff, Methansulfonsäure oder Oxalsäure ist.

4. Herstellungsverfahren gemäss Anspruch 1 oder 2, wobei die Säure (2) (S)-10-Camphersulfonsäure oder D-Weinsäure ist.

5. Herstellungsverfahren gemäss irgendeinem der Ansprüche 1 bis 4, wobei das 8-substituierte (1S,6R)-cis-7,9-Dioxo-2,8-diazabicyclo[4.3.0]nonan-Derivat der Formel (1) durch Umsetzen eines 6-substituierten 2,3,4,5,6,7-Hexahydro-1H-pyrrolo[3,4-b]pyridin-5,7-dion-Derivats der nachstehenden Formel (6): 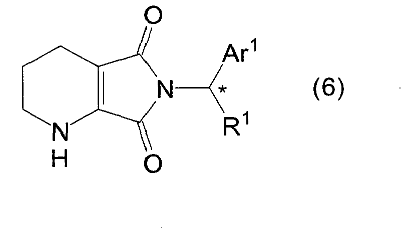
worin * einen asymmetrischen Kohlenstoff bezeichnet; Ar¹ eine Phenylgruppe ist; R¹ eine Methylgruppe ist,
oder eines Salzes davon mit Wasserstoff in Gegenwart eines Metallkatalysators hergestellt wird.

6. Verfahren zur Herstellung von (1S,6S)-cis-2,8-Diazabicyclo[4.3.0]nonan der nachstehenden Formel (5) : 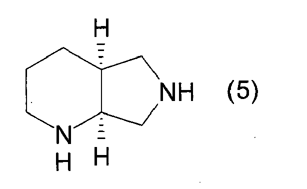 oder des Salzes davon, umfassend die Schritte:
optionales Neutralisieren des festen 8-substituierten (1S,6R)-cis-7,9-Dioxo-2,8-diazabicyclo[4.3.0]nonan-Derivats der nachstehenden Formel (3): 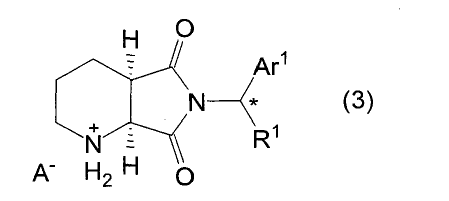
worin *, Ar¹, R¹ und A⁻ die gleichen wie oben sind,
hergestellt nach dem Verfahren gemäss irgendeinem der Ansprüche 1 bis 5,
und dann Umsetzen mit einem Reduktionsmittel zur Herstellung eines 8-substituierten (1S,6S)-cis-2,8-diazabicyclo[4.3.0]nonan-Derivats der nachstehenden Formel (4): 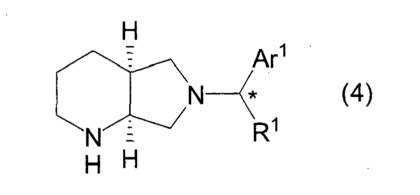
worin *, Ar¹ und R¹ die gleichen wie oben sind,
oder eines Salzes davon,
und anschliessendes Umsetzen mit Wasserstoff in Gegenwart eines Metallkatalysators zur Entfernung der Substituentengruppe am Stickstoffatom in der 8-Position.

7. Festes 8-substituiertes (1S,6R)-cis-7,9-Dioxo-2,8-diazabicyclo[4.3.0]nonan-Derivat der nachstehenden Formel (3): 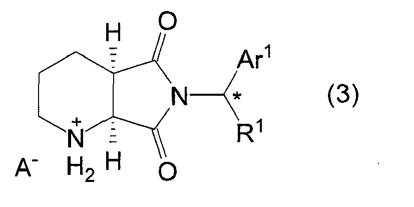
worin * einen asymmetrischen Kohlenstoff bezeichnet; Ar¹ eine gegebenenfalls substituierte C₆₋₂₀-Arylgruppe oder eine gegebenenfalls substituierte C₃₋₂₀-Heteroarylgruppe ist und R¹ eine gegebenenfalls substituierte C₁₋₂₀-Alkylgruppe ist.

8. Feststoff gemäss Anspruch 7, wobei Ar¹ eine Phenylgruppe ist, R¹ eine Methylgruppe ist und die absolute Konfiguration des asymmetrischen Kohlenstoffs in der Substituentengruppe am Stickstoffatom in der 8-Position S ist.

9. Feststoff gemäss Anspruch 7 oder 8, wobei A⁻ ein Chloridion, ein Hydrogensulfation, ein Bromidion, ein Methansulfonation oder ein Oxalation ist.

10. Feststoff gemäss Anspruch 7 oder 8, wobei A⁻ ein (S)-10-Camphersulfonation oder ein D-Tartration ist.

11. Feststoff gemäss Anspruch 7, wobei A⁻ ein Chloridion ist und der Feststoff ein Pulver-Röntgenbeugungsmuster mit spezifischen Peaks bei 4,5°, 14,5°, 18,2°, 21,1° und 22,8° in Winkelgraden von 2θ aufweist.

12. Feststoff gemäss Anspruch 7, wobei A⁻ ein Chloridion ist und der Feststoff ein Pulver-Röntgenbeugungsmuster mit spezifischen Peaks bei 4,5°, 9,0°, 13,9°, 14,5°, 18,2°, 19,6°, 21,1°, 22,8°, 23,9°, 24,9°, 27,5° und 28,6° in Winkelgraden von 2θ aufweist.

13. 6-substituiertes 2,3,4,5,6,7-Hexahydro-1H-pyrrolo-[3,4-b]pyridin-5,7-dion-Derivat der nachstehenden Formel (6); 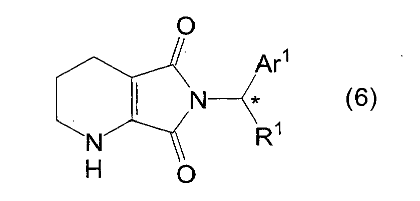
worin * einen asymmetrischen Kohlenstoff bezeichnet; Ar¹ eine Phenylgruppe ist; R¹ eine Methylgruppe ist,
oder ein Salz davon.

14. Verfahren zur Herstellung eines festen 8-substituierten (1R,6S)-cis-7,9-Dioxo-2,8-diazabicyclo[4.3.0]nonan-Derivats, umfassend einen Schritt, in dem aus einem 8-substituierten (1R,6S)-cis-7,9-Dioxo-2,8-diazabicyclo[4.3.0]nonan-Derivat der nachstehenden Formel (8): 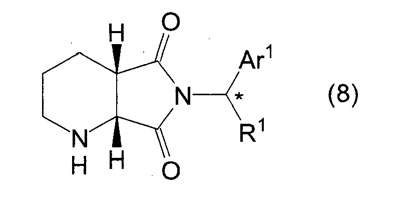
worin * einen asymmetrischen Kohlenstoff bezeichnet; Ar¹ eine gegebenenfalls substituierte C₆₋₂₀-Arylgruppe oder eine gegebenenfalls substituierte C₃₋₂₀-Heteroarylgruppe ist; R¹ eine gegebenenfalls substituierte C₁₋₂₀-Alkylgruppe ist; und worin ein 8-substituiertes (1S,6R)-cis-7,9-Dioxo-2,8-diazabicyclo[4.3.0]nonan-Derivat als Verunreinigung enthalten ist,
mit einer Säure der nachstehenden Formel (2):
H⁺A⁻ (2)
worin A⁻ ein Gegenanion ist,
ein Salz gebildet wird, um das feste 8-substituierte (1R,6S)-cis-7,9-Dioxo-2,8-diazabicyclo[4.3.0]nonan-Derivat der nachstehenden Formel (9) auszufällen: 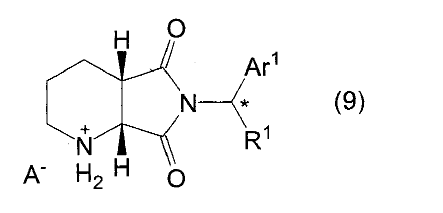
worin *, Ar¹, R¹ und A⁻ die gleichen wie oben sind.

## Revendications

1. Procédé de production d'un dérivé (1S,6R)-cis-7,9-dioxo-8-substitué-2,8-diazabicyclo[4.3.0]nonane solide, comprenant l'étape de formation d'un sel à partir d'un dérivé (1S,6R)-cis-7,9-dioxo-8-substitué-2,8-diazabicyclo[4.3.0]nonane représenté par la formule (1) suivante : 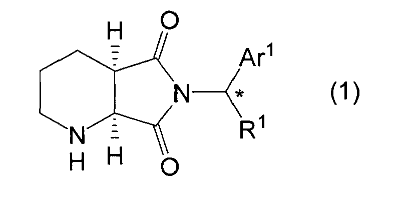
dans laquelle * indique un carbone asymétrique ; Ar¹ est un groupe aryle en C₆₋₂₀ éventuellement substitué ou un groupe hétéroaryle en C₃₋₂₀ éventuellement substitué ; R¹ est un groupe alkyle en C₁₋₂₀ éventuellement substitué ; et dans lequel un dérivé (1R,6S)-cis-7,9-dioxo-8-substitué-2,8-diazabicyclo[4.3.0]nonane est contenu sous forme d'impureté,
avec un acide représenté par la formule (2) suivante :
H⁺A⁻ (2)
dans laquelle A⁻ est un contre-anion,
pour précipiter le dérivé (1S,6R)-cis-7,9-dioxo-8-substitué-2,8-diazabicyclo[4.3.0]nonane solide représenté par la formule (3) suivante : 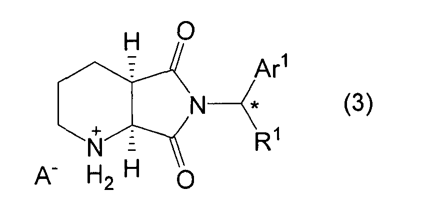
dans laquelle *, Ar¹, R¹ et A⁻ sont les mêmes que ci-dessus.

2. Procédé de production selon la revendication 1, dans lequel Ar¹ est un groupe phényle, R¹ est un groupe méthyle, et une configuration absolue du carbone asymétrique est S, dans le groupe substituant sur l'atome d'azote en position 8.

3. Procédé de production selon la revendication 1 ou 2, dans lequel l'acide (2) est le chlorure d'hydrogène, l'acide sulfurique, le bromure d'hydrogène, l'acide méthanesulfonique ou l'acide oxalique.

4. Procédé de production selon la revendication 1 ou 2, dans lequel l'acide (2) est l'acide (S)-10-camphosulfonique ou l'acide D-tartrique.

5. Procédé de production selon l'une quelconque des revendications 1 à 4, dans lequel le dérivé (1S,6R)-cis-7,9-dioxo-8-substitué-2,8-diazabicyclo[4.3.0]nonane représenté par la formule (1) est produit par la réaction d'un dérivé 6-substitué-2,3,4,5,6,7-hexahydro-1H-pyrrolo[3,4-b]pyridine-5,7-dione représenté par la formule (6) suivante : 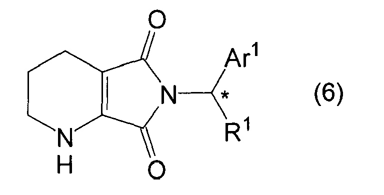
dans laquelle * indique un carbone asymétrique ; Ar¹ est un groupe phényle ; R¹ est un groupe méthyle,
ou le sel de celui-ci avec de l'hydrogène en présence d'un catalyseur métallique.

6. Procédé de production du (1S,6S)-cis-2,8-diazabicyclo[4.3.0]nonane représenté par la formule (5) suivante : 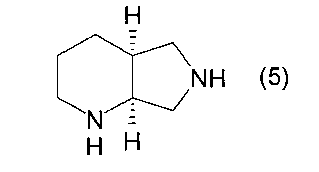
ou le sel de celui-ci, comprenant les étapes de
éventuellement neutralisation du dérivé (1S,6R)-cis-7,9-dioxo-8-substitué-2,8-diazabicyclo[4.3.0]nonane solide représenté par la formule (3) suivante : 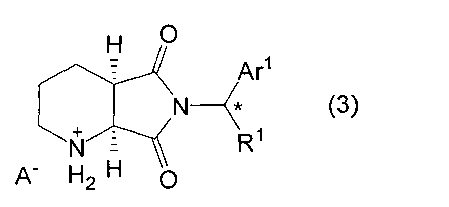
dans laquelle *, Ar¹, R¹ et A⁻ sont les mêmes que ci-dessus,
produit par le procédé selon l'une quelconque des revendications 1 à 5,
puis la réaction d'un agent réducteur pour produire un dérivé (1S,6S)-cis-8-substitué-2,8-diazabicyclo[4.3.0]nonane représenté par la formule (4) suivante : 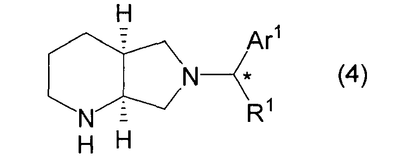
dans laquelle *, Ar¹ et R¹ sont les mêmes que ci-dessus, ou le sel de celui-ci,
et par la suite, la réaction d'hydrogène en présence d'un catalyseur métallique pour éliminer le groupe substituant sur l'atome d'azote en position 8.

7. Dérivé (1S,6R)-cis-7,9-dioxo-8-substitué-2,8-diazabicyclo[4.3.0]nonane solide représenté par la formule (3) suivante : 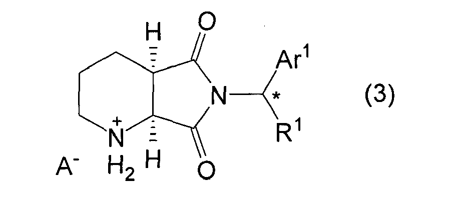
dans laquelle * indique un carbone asymétrique ; Ar¹ est un groupe aryle en C₆₋₂₀ éventuellement substitué ou un groupe hétéroaryle en C₃₋₂₀ éventuellement substitué ; R¹ est un groupe alkyle en C₁₋₂₀ éventuellement substitué.

8. Solide selon la revendication 7, dans lequel Ar¹ est un groupe phényle, R¹ est un groupe méthyle, et une configuration absolue du carbone asymétrique est S, dans le groupe substituant sur l'atome d'azote en position 8.

9. Solide selon la revendication 7 ou 8, dans lequel A⁻ est un ion chlorure, un ion hydrogénosulfate, un ion bromure, un ion méthanesulfonate ou un ion oxalate.

10. Solide selon la revendication 7 ou 8, dans lequel A⁻ est un ion (S)-10-camphosulfonate ou un ion D-tartrate.

11. Solide selon la revendication 7, dans lequel A⁻ est un ion chlorure et le solide présente un schéma de diffraction des rayons X sur poudre ayant des pics spécifiques à 4,5°, 14,5°, 18,2°, 21,1° et 22,8° en degrés 2θ.

12. Solide selon la revendication 7, dans lequel A⁻ est un ion chlorure et le solide présente un schéma de diffraction des rayons X sur poudre ayant des pics spécifiques à 4,5°, 9,0°, 13,9°, 14,5°, 18,2°, 19,6°, 21,1°, 22,8°, 23,9°, 24,9°, 27,5° et 28,6° en degrés 2θ.

13. Dérivé 6-substitué-2,3,4,5,6,7-hexahydro-1H-pyrrolo[3,4-b]pyridine-5,7-dione représenté par la formule (6) suivante : 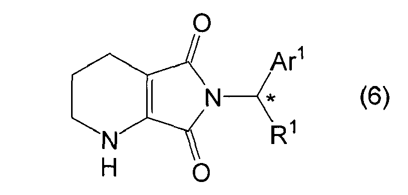
dans laquelle * indique un carbone asymétrique ; Ar¹ est un groupe phényle ; R¹ est un groupe méthyle,
ou le sel de celui-ci.

14. Procédé de production d'un dérivé (1R,6S)-cis-7,9-dioxo-8-substitué-2,8-diazabicyclo[4.3.0]nonane solide, comprenant l'étape de formation d'un sel à partir d'un dérivé (1R,6S)-cis-7,9-dioxo-8-substitué-2,8-diazabicyclo[4.3.0]nonane représenté par la formule (8) suivante : 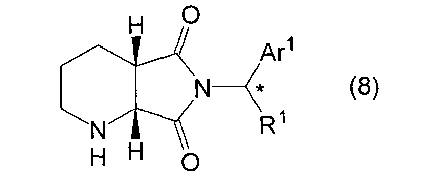
dans laquelle * indique un carbone asymétrique ; Ar¹ est un groupe aryle en C₆₋₂₀ éventuellement substitué ou un groupe hétéroaryle en C₃₋₂₀ éventuellement substitué ; R¹ est un groupe alkyle en C₁₋₂₀ éventuellement substitué ; et dans lequel un dérivé (1S,6R)-cis-7,9-dioxo-8-substitué-2,8-diazabicyclo[4.3.0]nonane est contenu sous forme d'impureté,
avec un acide représenté par la formule (2) suivante :
H⁺A⁻ (2)
dans laquelle A⁻ est un contre-anion,
pour précipiter le dérivé (1R,6S)-cis-7,9-dioxo-8-substitué-2,8-diazabicyclo[4.3.0]nonane solide représenté par la formule (9) suivante : 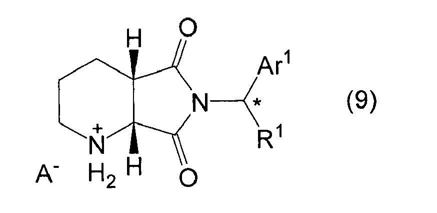
dans laquelle *, Ar¹, R¹ et A⁻ sont les mêmes que ci-dessus.
